(19) Europäisches Patentamt
European Patent Office
Office européen des brevets

(11) EP 1 142 587 A1

(12) EUROPEAN PATENT APPLICATION
published in accordance with Art. 158(3) EPC

(43) Date of publication:
**10.10.2001 Bulletin 2001/41**

(21) Application number: **99959951.7**

(22) Date of filing: **21.12.1999**

(51) Int Cl.[7]: **A61K 45/00**, A61K 31/438, A61K 31/4409, C07D 401/14, C07D 401/04, A61P 25/04, C07D 491/107, C07D 471/10

(86) International application number:
**PCT/JP99/07191**

(87) International publication number:
**WO 00/38720 (06.07.2000 Gazette 2000/27)**

(84) Designated Contracting States:
**AT BE CH CY DE DK ES FI FR GB GR IE IT LI LU MC NL PT SE**
Designated Extension States:
**AL LT LV MK RO SI**

(30) Priority: **24.12.1998 JP 36736698**
**18.05.1999 JP 13681299**

(71) Applicant: **Meiji Seika Kaisha, Ltd.**
**Tokyo 104-8002 (JP)**

(72) Inventors:
- **Akiyama, Yoshihisa, Pharm. Res. Ctr. Yokohama-shi, Kanagawa 222-8567 (JP)**
- **Kudoi, Toshiaki, Pharm. Res. Ctr. Yokohama-shi, Kanagawa 222-8567 (JP)**
- **Mori, Tomohisa, Pharm. Res. Ctr. Yokohama-shi, Kanagawa 222-8567 (JP)**
- **Asai, Kenji, Pharm. Res. Ctr. Yokohama-shi, Kanagawa 222-8567 (JP)**
- **Miike, Naoko, Pharm. Res. Ctr. Yokohama-shi, Kanagawa 222-8567 (JP)**
- **Yanagisawa, Yumiko, Pharm. Res. Ctr. Yokoham-shi, KKanagawa 222-8567 (JP)**
- **Watanabe, Takashi, Pharm. Res. Ctr. Yokohama-shi, Kanagawa 222-8567 (JP)**
- **Tsushima, Masaki, Pharm. Res. Ctr. Yokohama-shi, Kanagawa 222-8567 (JP)**
- **Hiranuma, Toyokazu, Pharm. Res. Ctr. Yokohama-shi, Kanagawa 222-8567 (JP)**

(74) Representative: **Hall, Marina**
**Elkington and Fife**
**Prospect House,**
**8 Pembroke Road**
**Sevenoaks, Kent TN13 1XR (GB)**

# (54) REMEDIES FOR PAIN

(57) The present invention provides a pain control agent, which contains, as an active ingredient, a compound having both μ opioid receptor agonist activity and dopamine D2 receptor antagonist activity. The compound having both of these activities exerts a potent morphine-like analgetic effect and causes no psychological dependence, and even regulates side effects.

In particular, a novel compound represented by general formula (I) or a pharmacologically acceptable salt thereof has both μ opioid receptor agonist activity and dopamine D2 receptor antagonist activity, and is useful as pain control agent with regulated side effects.

EP 1 142 587 A1

## Description

### TECHNICAL FIELD

The present invention relates to pain control agent, which shows a potent morphine-like analgetic effect and regulates side effects. Moreover, the present invention relates to a remedy, which comprises a novel compound having a potent analgetic effect or a pharmacologically acceptable salt thereof.

### BACKGROUND ART

As a potent analgetic agent, opioid analgetic agents (pain control agent) such as morphine have been used for pain control agent since long ago. Whereas morphine, which has a strong opioid analgetic agent shows effectiveness against strong pain by acting on μ opioid receptors (agonist activity), there is a problem that its side effects such as nausea and neurologic manifestation including hallucination and derangement. These side effects make difficult for pain control agent, resulting in a reduction of the effectiveness of pain treatment. In addition, it was also well known that a strong opioid analgetic agent such as morphine forms psychological dependence, causing serious problems.
Other weak opioid analgetic agents such as pentazocine and buprenorphine do not have effectiveness on strong pain. These agents induce psychological and physiological dependence, although these effects are rather weak. In spite of this fact, since these agents are not designated as narcotics at this moment, and so the management of agents is not necessarily adequate, the theft, abuse etc. of the agents cause social problems. In addition, it was also known that the use of pentazocine and buprenorphine produces the same side effects of morphine, although these effects are rather weak.
Psychological dependence, nausea, hallucination and derangement, which are side effects induced by the use of a strong opioid analgetics, are considered to be associated with a activation of dopamine D2 receptor. Psychological dependence of morphine is considered to be caused by a dopamine release from nucleus accumbens which might be related to activatin of μ opioid receptors, and this phenomenon is inhibited by dopamine D1 and dopamine D2 receptor antagonists. Nausea and vomition are also considered to be caused by a dopamine release which may be mediated through activation of μ receptors, and these symptoms are actually treated with dopamine D2 receptor antagonists such as haloperidol.
Through previous studies with animal experiments, a large number of reports demonstrated that dopamine releasing effects in the brain are involved in the formation of psychological dependence with many kinds of dependence-forming agents, and that dopamine D2 receptor antagonists inhibit psychological dependence induced by such agents. I has been reported that psychological dependence of morphine is attenuated in dopamine D2 receptor knockout mice (*Nature*, 586-589 (1997)). However, the clinical use of dopamine D2 antagonists together with morphine for the purpose of inhibiting the formation of psychological dependence is still unknown, and there are also no reports regarding the degree of a dopamine D2 antagonist action necessary to inhibit such induction of psychological dependence.
Against nausea and neurologic manifestations such as hallucination and derangement, which are side effects of morphine, dopamine D2 receptor antagonist including haloperidol or the like have clinically been used with morphine, but the combined use of many kinds of agents are not medically preferable considering compliance and QOL of patients. Furthermore, there are actually no reports regarding the degree of a dopamine D2 antagonist action required to inhibit nausea and neurologic manifestation as well as induction of psychological dependence.
Against this backdrop, apart from the combined use of morphine as pain control agent and other agents inhibiting side effects, a novel pain control agent, which shows, with a single agent, a potent analgetic effect at least equivalent to that of morphine, causing no psychological dependence and regulating side effects is desired.
A compound structurally associated with the compound of general formula (I) of the present invention (spiro [isobenzofuran-1(3H)-4'-piperidine]-3-imine or spiro[isobenzofuran-1(3H)-4'-piperidine]-3-on derivatives) is described in WO94/29309, US5627196, US5614523, EP0722941, WO98/08835, WO98/20010 and WO94/13696. And a method for synthesizing spiro[isobenzofuran-1(3H)-4'-piperidine]-3-imine or spiro[isobenzofuran-1(3H)-4'-piperidine]-3-on is disclosed in *J.Org.Chem.*, *41,* 2628 (1976), and derivatives of these compounds are disclosed in *J.Org.Chem.*, *47,* 2681(1982), *Can.J.Chem.*, 64, 621(1986), *J.Med.Chem.*, *37,* 364(1994), *J.Med.Chem.*, 35, 2033(1992), *T.L.*, 39, 2331 (1998), *T.L.*, 24, 2649(1983), *T.L.*, 39, 2965(1998) and so on.
Compounds of general formulas (II) and (III) are disclosed in US 3155669, US 3155670, US 3318900, US 3345364 and so on.
However, there are no reports with regard to μ opioid receptor affinity and analgetic activity, and dopamine D2 receptor affinity of these compounds.
An object of the present invention is to provide a novel pain control agent, which overcomes the above problems. That is to say, the object of the present invention is to provide a pain control agent having, with a single agent, a potent analgetic effect and regulating the expression of side effects. Furthermore, another object of the present

invention is to provide a remedy comprising a novel compound with the above characteristics or pharmacologically acceptable salts thereof.

DISCLOSURE OF THE INVENTION

We have analyzed various compounds to achieve the above objects, and found that a novel compound having both μ opioid receptor agonist activity and dopamine D2 receptor antagonist activity. The compound having both these activities has not been reported yet. We have found that the above compound exhibits a potent analgetic effect, and dose not induce a psychological dependence and locomotor activity promoting effect, such as is exhibited by strong opioids.

The term "a compound having μ opioid receptor agonist activity" is used herein to mean a compound showing a binding affinity in a μ opioid receptor membrane binding test, and also showing analgetic activity using hot plate test. Specifically, (1) the binding affinity can be defined as the strength of test substance to substitute for binding of [$^3$H]-DAMGO (which denotes Ki value obtained according to the formula of Cheng and Prusoff, after determining the $IC_{50}$ value of each compound by a nonlinear method of least squares regression analysis), in the presence of a μ receptor membrane fraction prepared from the rat forebrain and a radioactive ligand (1nM (final concentration) [$^3$H]-DAMGO ([D-Ala$^2$,N-Me-Phe$^4$,Gly-ol]-enkephalin)) labeling the μ receptor; and (2) analgetic activity can be defined as an effect that a test substance administered-mouse placed on a 52°C hot plate shows avoidance behavior against thermal stimulation (which refers to $ED_{50}$ value calculated from the dose response of each compound by a least square method).

In the present invention, where a compound shows a binding affinity as above, a compound with a strong affinity, whose Ki value to a μ opioid receptor is less than 50 nM, is particularly preferable.

The term "a compound having dopamine D2 receptor antagonist activity" is used herein to mean a compound showing a binding affinity in a D2 receptor membrane binding test, and also showing inhibiting effect to apomorphine-induced climbing behavior. Specifically, (1) the binding affinity can be defined as the strength of test substance to substitute for the bond of [$^3$H]-spiperone (which denotes Ki value obtained according to the formula of Cheng and Prusoff, after determining the $IC_{50}$ value of each compound by a nonlinear method of least squares regression analysis), in the presence of a dopamine D2 receptor membrane fraction prepared from the rat corpus striatum and a radioactive ligand (0.1nM (final concentration) of [$^3$H]-spiperone) labeling the D2 receptor; and (2) inhibiting activity to the apomorphine-induced climbing behavior can be defined as the strength to inhibit climbing behavior of a test substance administered-mouse (which refers to $ED_{50}$ value calculated from the dose response of each compound by a least square method), which shows characteristic climbing behavior caused by the administration of apomorphine.

In order to specifically indicate the relative activity ratio between μ opioid receptor agonist activity and dopamine D2 receptor antagonist activity, the activity ratio herein is defined as the ratio between the above-stated binding affinity of a compound in the μ opioid receptor membrane binding test and the above-stated binding affinity of the same compound in the D2 receptor membrane binding test.

After thorough studies based on the above definitions, we found that, a compound having both μ opioid receptor agonist activity and dopamine D2 receptor antagonist activity, which activity ratio between μ opioid receptor agonist activity and dopamine D2 receptor antagonist activity is preferably 1 : 1 to 1 : 150, more preferably 1 : 10 to 1 : 30, shows strong analgetic activity, regulating side effects, and can be used as a useful pain control agent, with a single agent; thereby completing the present invention.

That is to say, the present invention comprises the following features:

1. A pain control agent, which contains, as an active ingredient, a compound having both μ opioid receptor agonist activity and dopamine D2 receptor antagonist activity.

2. The pain control agent according to the above 1, wherein the ratio between μ opioid receptor agonist activity and dopamine D2 receptor antagonist activity is 1 : 1 to 1 : 150.

3. The pain control agent according to the above 1 or 2, wherein the active ingredient is a compound selected from a group consisting of the following general formulas (I) to (III) or pharmacologically acceptable salts thereof:

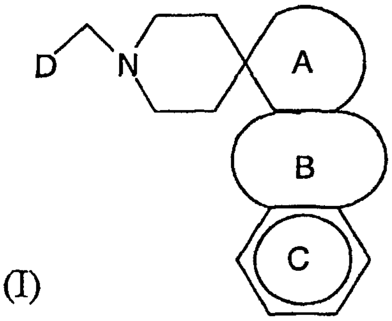

(I)

wherein

ring A represents a saturated 5- or 6-membered ring, which may be substituted and may comprise a heteroatom(s) selected from S, N and O,
ring B represents a 5- or 6-membered aromatic ring, which may comprise one heteroatom selected from N and O,
ring C represents a benzene ring or a pyridine ring, which may be substituted, and
ring D represents an aromatic ring, which may be substituted and may comprise a heteroatom(s) selected from S, N and O;

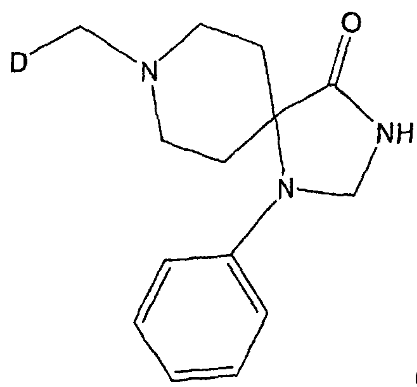

(II)

wherein
D represents an aromatic ring, which may be substituted and may comprise a heteroatom(s) selected from S, N and O; and

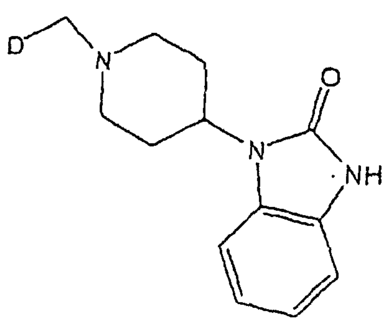

(III)

wherein
D represents an aromatic ring, which may be substituted and may comprise a heteroatom(s) selected from S, N and O.

4. A compound shown in the following general formula (I) or a pharmacologically acceptable salt thereof:

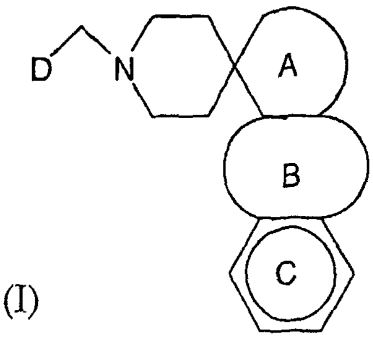

(I)

wherein

ring A represents a saturated 5- or 6-membered ring, which may be substituted and may comprise a heteroatom(s) selected from S, N and O,
ring B represents a 5- or 6-membered aromatic ring, which may comprise one heteroatom selected from N and O,
ring C represents a benzene ring or a pyridine ring, which may be substituted, and
ring D represents an aromatic ring, which may be substituted and may comprise a heteroatom(s) selected from S, N and O.

5. The compound or pharmacologically acceptable salts thereof according to the above 4, wherein

ring A is shown in any one of the following general formulas (a) to (e):

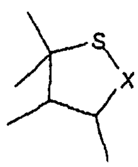

(a)

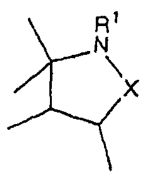

(b)

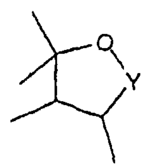

(c)

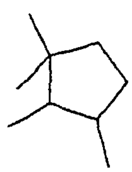

(d)

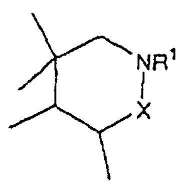

(e)

wherein $R^1$ is H or lower alkyl,

X is $CH_2$ or C=O, and
Y is $CH_2$, C=O or C=NH;

ring B is shown in any one of the following general formulas (f) to (h):

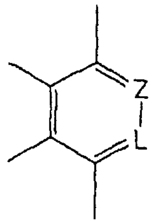

(f)

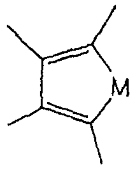

(g)

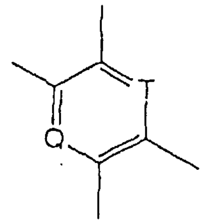

(h)

wherein
either one of Z and L is N and the other is CH, or both are CH,
M is NH or O, and
either one of Q and T is N and the other is CH, or both are CH; and
D is shown in any one of the following general formulas (i) to (n):

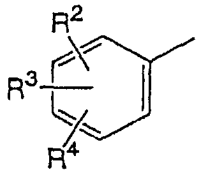

(i)

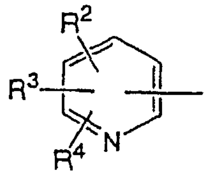

(j)

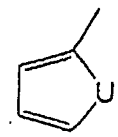

(k)

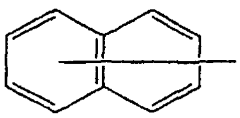

(l)

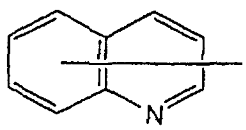

(m)

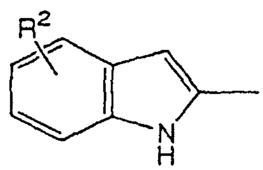

(n)

wherein
each of $R^2$, $R^3$ and $R^4$ represents independently hydrogen, lower alkyl, lower alkoxy, lower alkylthio, lower alkoxycarbonyl, cyano, halogen, hydroxy, carbamoyl, nitro or amino, and
U represents S, O or $NR^5$ ($R^5$ is H or lower alkyl).

6. The compound or pharmacologically acceptable salt thereof according to the above 5, wherein ring C is benzene.

7. The compound or pharmacologically acceptable salt thereof according to the above 6, wherein

ring A is (c), wherein Y is C=O or C=NH;
ring B is (f), wherein both of Z and L are CH; and
D is (i) or (j), wherein each of $R^2$, $R^3$ and $R^4$ is independently chlorine, fluorine, hydrogen or methoxy.

8. A compound shown in the following general formula (IV) or pharmacologically acceptable salt thereof:

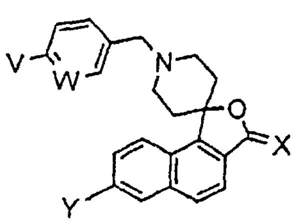

(IV)

wherein V is hydrogen, chlorine, fluorine or methoxy; W is nitrogen or CH; X is NH or O; and Y is hydrogen, hydroxyl or methoxy.

9. A compound shown in the following general formula (V) or pharmacologically acceptable salt thereof:

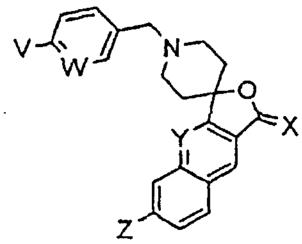

(V)

wherein V is hydrogen or chlorine; W is nitrogen or CH; X is NH or O; Y is CH or nitrogen, and Z is hydrogen or methoxy.

10. A compound shown in the following general formula (VI) or pharmacologically acceptable salt thereof:

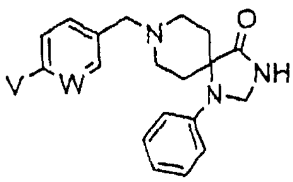

(VI)

wherein V is hydrogen, chlorine, bromine, methyl or methoxy; W is nitrogen or CH.

11. A compound shown in the following general formula (VII) or pharmacologically acceptable salt thereof:

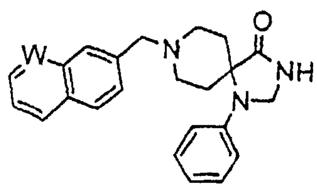

(VII)

wherein W is nitrogen or CH.

12. A compound shown in the following general formula (VIII) or pharmacologically acceptable salt thereof:

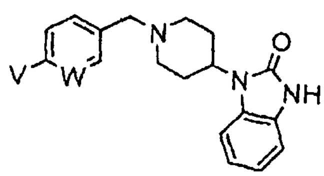

(VIII)

wherein V is chlorine or methoxy, and W is nitrogen or CH.

13. A compound shown in the following general formula (IX) or pharmacologically acceptable salt thereof:

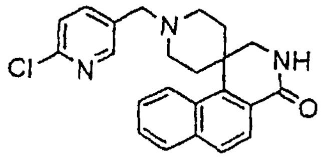

(IX)

14. A remedy containing the compound or pharmacologically acceptable salt thereof according to any one of the above 4 to 13.

15. Pain control agent containing the compound or pharmacologically acceptable salt thereof according to any one of the above 4 to 13.

This specification includes part or all of the contents as disclosed in the specifications and/or drawings of Japanese Patent Application Nos. 10-367366 and 11-136812, which are priority documents of the present application.

## BRIEF DESCRIPTION OF THE DRAWINGS

Figure 1 shows the position palatability conditioned by the compound of Example 14 and morphine.
Figure 2 shows the position palatability conditioned by the compound of Example 16 and morphine.

## EMBODIMENTS OF THE INVENTION

Generally, the use of a compound having μ opioid receptor agonist activity as pain control agent is known. However, novel compounds of general formulas (I) to (III) of the present invention and a pharmacologically acceptable salt thereof are characterized in that those have both a μ opioid receptor agonist activity and dopamine D2 receptor antagonist activity. It became clear that the above compounds show a potent analgetic effect, though it does not cause psychological dependence and locomotor activity promoting effect as does a strong opioid.
First, the novel compounds of the present invention are described further in detail.
The term "lower alkyl" as a group or a part of group is used herein to mean linear or branched alkyl chain containing 1 to 6, preferably 1 to 4 carbon atoms, and examples of such alkyl include methyl, ethyl, isopropyl, isobutyl, t-butyl and so on. The term "halogen atom" means fluorine, chlorine, bromine and iodine. The term "alkoxy" herein means alkyloxy which may be substituted, preferably alkyloxy containing 1 to 4 carbon atoms, and the examples include linear or branched chain alkyloxy such as methoxy, ethoxy and isopropyloxy, or cyclic alkyloxy such as tetrahydropyranyloxy.
In general formula (I), ring A represents a saturated 5- or 6-membered ring, which may be substituted and may comprise a heteroatom(s) selected from S, N and O, and examples of the ring include those shown in the following general formulas (a) to (e):

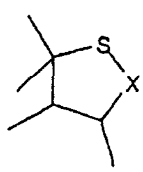

(a)

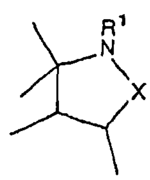

(b)

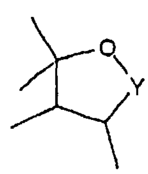

(c)

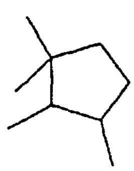

(d)

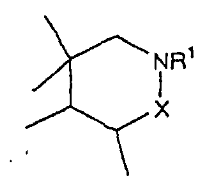

(e)

wherein $R^1$ is H or lower alkyl.
A preferred example includes a dihydrofuran group shown in the above general formula (c), and more preferably includes dihydrofuranone wherein Y is C=O, or dihydrofuranimine wherein Y is C=NH.

Ring B represents a 5- or 6-membered aromatic ring, which may comprise a heteroatom selected from N and O, and examples of the ring include those shown in the following general formulas (f) to (h):

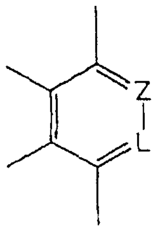
(f)

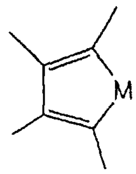
(g)

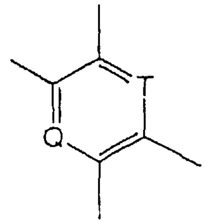
(h)

13

Preferred examples include a benzene ring or a pyridine ring, which are shown in the above general formula (f), and more preferably includes a benzene ring wherein both Z and L concurrently represent CH.

Ring C represents a benzene ring or a pyridine ring, which may be substituted, and a preferred example includes a benzene ring.

D represents an aromatic ring, which may be substituted and may comprise a heteroatom(s) selected from S, N and O, and examples of the ring include aromatic rings shown in the following general formulas (i) to (n):

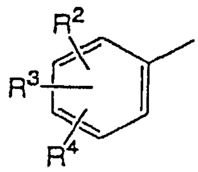
(i)

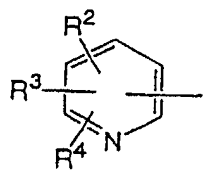
(j)

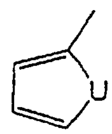
(k)

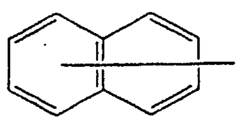
(l)

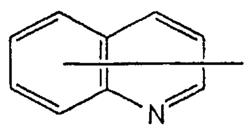
(m)

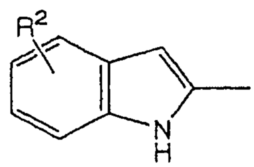
(n)

Preferred examples include a benzene ring, a substituted benzene ring, a pyridine ring or a substituted pyridine ring, which are shown in the above general formula (i) or (j).

In the above formulas, each of substituents $R^2$, $R^3$ and $R^4$ represents independently hydrogen, lower alkyl, lower alkoxy, lower alkylthio, lower alkoxycarbonyl, cyano, halogen, hydroxy, carbamoyl, nitro or amino. Lower alkylthio herein represents linear or branched alkylthio containing 1 to 4 carbon atoms, such as methylthio, ethylthio and isopropylthio. Lower alkoxycarbonyl herein represents alkoxycarbonyl containing 1 to 4 carbon atoms such as methyloxycarbonyl, ethyloxycarbonyl and isopropyloxycarbonyl. $R^2$, $R^3$ and $R^4$ can independently substitute for any of hydrogen atoms on a ring, i.e. it may be that none of the hydrogen atoms are substituted by any substituent other than a hydrogen atom, or one or more hydrogen atoms may be identically or differently substituted by any substituent other than a hydrogen atom at one or more points on the ring.

A preferred example of the compound of the present invention includes a compound, wherein, in the above general formula (I), ring A is (c), Y is C=O or C=NH; ring B is (f), both Z and L are CH; ring C is a benzene ring; and D is (i) or (j), each of $R^2$, $R^3$ and $R^4$ is chlorine, fluorine, hydrogen or methoxy.

A particularly preferable group of compounds of the present invention includes a compound shown in the following general formula (IV), which belongs to the compounds of general formula (I), or pharmacologically acceptable salt thereof:

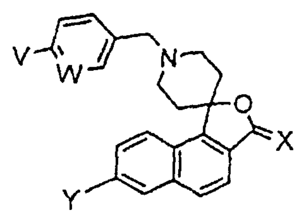

(IV)

wherein V is hydrogen, chlorine, fluorine or methoxy; W is nitrogen or CH; X is NH or O; and Y is hydrogen, hydroxyl or methoxy.

Another particularly preferable group of compounds of the present invention includes a compound shown in the following general formula (V), which belongs to the compounds of general formula (I), or pharmacologically acceptable salt thereof:

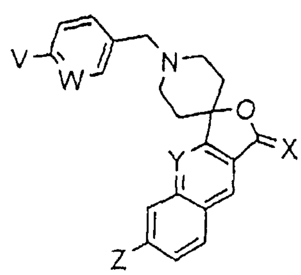

(V)

wherein V is hydrogen or chlorine; W is nitrogen or CH; X is NH or O; Y is CH or nitrogen, and Z is hydrogen or methoxy.

A further particularly preferable group of compounds of the present invention includes a compound shown in the following general formula (IX), which belongs to the compounds of general formula (I), or pharmacologically acceptable salt thereof:

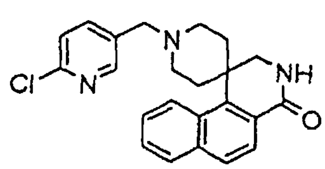

(IX)

Particularly preferable compounds of the present invention are described, and specifically named those below, however, a person skilled in the art will realize that, in the case of a complicated organic compound, a plurality of terms may exist for an identical compound depending on the nomenclature applied. Therefore, the nomenclature used in the present specification is described below. The compound shown in the following structural formula is herein referred as 1'-benzyl(benzo[g]1,3-dihydroisobenzofuran-1-spiro-4'-piperidine)-3-on.

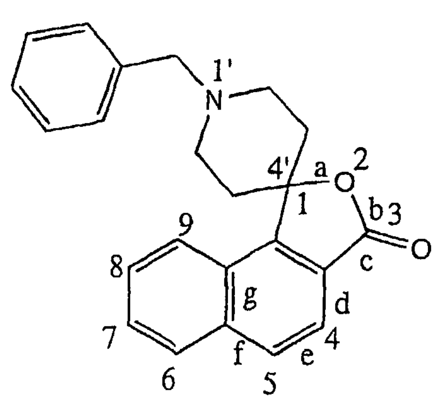

In Japanese Patent Application No. 10-367366, which is a priority application of the present application, the spirohydrocarbon number, heterocyclic ring atom number and the characteristic ring number of this compound are shown as follows, and the compound is referred as 1'-benzyl-benzo[c]spiro[benzodihydrofuran-1(3H), 4'-piperidine]-3-on.

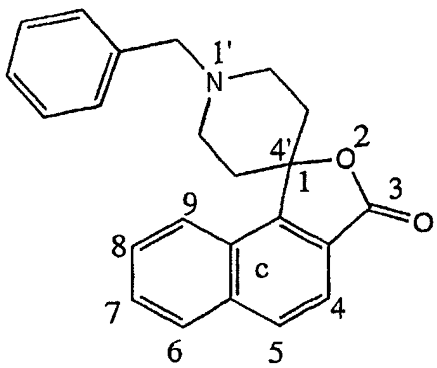

In Japanese Patent Application No. 11-136812, which is also a priority application of the present application, the spirohydrocarbon number, heterocyclic ring atom number and the characteristic ring number of this compound are shown as follows, and the compound is referred as 14-benzylspiro[hydrobenzo[e]isobenzofuran-1, 4'-piperidine]-3-on.

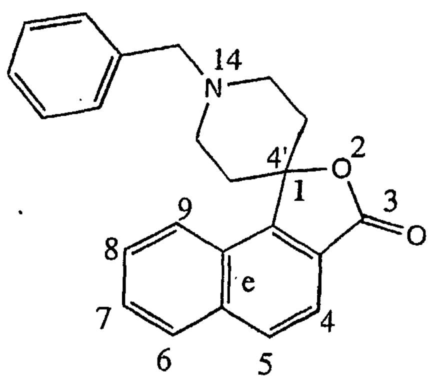

With this being the situation, in respect of compounds described in priority documents to the present application, the compound name used in the priority application is also written as an alias name in parentheses.

Examples of a preferable group of compounds of the present invention include,

**1'-(6-chloro-3-pyridyl)methyl(benzo[g]1,3-dihydroisobenzofuran-1-spiro-4'-piperidine)-3-on** (alias: 1'-(2-chloropyridyl)-benzo[c]spiro[benzodihydrofuran-1(3H),4'-piperidine]-3-on (Japanese Patent Application No. 10-367366), or 14-[(6-chloro-3-pyridyl)methyl]spiro[hydrobenzo[e]isobenzofuran-1,4'-piperidine]-3-on (Japanese Patent Application No. 11-136812));

**1'-benzyl(benzo[g]1,3-dihydroisobenzofuran-1-spiro-4'-piperidine)-3-on** (alias: 1'-benzyl-benzo[c]spiro[benzodihydrofuran-1(3H),4'-piperidine]-3-on (Japanese Patent Application No. 10-367366), or 14-benzylspiro[hydrobenzo[e]isobenzofuran-1,4'-piperidine]-3-on (Japanese Patent Application No. 11-136812));

**1'-(6-chloro-3-pyridyl)methyl(benzo[g]1,3-dihydroisobenzofuran-1-spiro-4'-piperidine)-3-imine** (alias: 1'-(2-chloropyridyl)-benzo[c]spiro[benzodihydrofuran-1(3H),4'-piperidine]-3-imine (Japanese Patent Application No. 10-367366), or 14-[(6-chloro-3-pyridyl)methyl]spiro[hydrobenzo[e]isobenzofuran-1,4'-piperidine]-3-imine (Japanese Patent Application No. 11-136812));

**1'-benzyl(benzo[g]1,3-dihydroisobenzofuran-1-spiro-4'-piperidine)-3-imine** (alias: 1'-benzyl-benzo[c]spiro[benzodihydrofuran-1(3H),4'-piperidine]-3-imine (Japanese Patent Application No. 10-367366), or 14-benzylspiro[hydrobenzo[e]isobenzofuran-1,4'-piperidine]-3-imine (Japanese Patent Application No. 11-136812));

**1'-(4-fluorobenzyl)(benzo[g]1,3-dihydroisobenzofuran-1-spiro-4'-piperidine)-3-imine** (alias: 1'-(4-fluorobenzyl)-benzo[c]spiro[benzodihydrofuran-1(3H),4'-piperidine]-3-imine (Japanese Patent Application No. 10-367366), or 14-(4-fluorobenzyl)spiro[hydrobenzo[e]isobenzofuran-1,4'-piperidine]-3-imine (Japanese Patent Application No. 11-136812));

**1'-(4-chlorobenzyl)(benzo[g]1,3-dihydroisobenzofuran-1-spiro-4'-piperidine)-3-imine** (alias: 1'-(4-chlorobenzyl)-benzo[c]spiro[benzodihydrofuran-1(3H),4'-piperidine]-3-imine (Japanese Patent Application No. 10-367366), or 14-(4-- chlorobenzyl)spiro[hydrobenzo[e]isobenzofuran-1,4'-piperidine]-3-imine (Japanese Patent Application No. 11-136812));

**1'-(4-methoxybenzyl) (benzo[g]1,3-dihydroisobenzofuran-1-spiro-4'-piperidine)-3-imine** (alias: 1'-(4-methoxybenzyl)-benzo[*c*]spiro[benzodihydrofuran-1(3H),4'-piperidine]-3-imine (Japanese Patent Application No. 10-367366), or 14-(4-methoxybenzyl)spiro[hydrobenzo[e]isobenzofuran-1,4'-piperidine]-3-imine (Japanese Patent Application No. 11-136812));

**1'-(4-fluorobenzyl)(benzo[g]1,3-dihydroisobenzofuran-1-spiro-4'-piperidine)-3-on** (alias: 1'-(4-fluorobenzyl)-benzo[c]spiro[benzodihydrofuran-1(3H),4'-piperidine]-3-on (Japanese Patent Application No. 10-367366), or 14-(4-fluorobenzyl)spiro[hydrobenzo[e]isobenzofuran-1,4'-piperidine]-3-on (Japanese Patent Application No. 11-136812));

**1'-(4-chlorobenzyl)(benzo[g]1,3-dihydroisobenzofuran-1-spiro-4'-piperidine)-3-on** (alias: 1'-(4-chlorobenzyl)-benzo[c]spiro[benzodihydrofuran-1(3H),4'-piperidine]-3-on (Japanese Patent Application No. 10-367366), or 14-(4-chlorobenzyl)spiro[hydrobenzo[e]isobenzofuran-1,4'-piperidine]-3-on (Japanese Patent Application No. 11-136812));

**1'-(4-methoxybenzyl)(benzo[g]1,3-dihydroisobenzofuran-1-spiro-4'-piperidine)-3-imine** (alias: 1'-(4-methoxybenzyl)-benzo[c]spiro[benzodihydrofuran-1(3H),4'-piperidine]-3-on (Japanese Patent Application No. 10-367366), or 14-(4-methoxybenzyl)spiro[hydrobenzo[e]isobenzofuran-1,4'-piperidine]-3-on (Japanese Patent Application No. 11-136812));

**1'-([(6-chloro-3-pyridyl)methyl](benzo[h]1,2,3-trihydroisoquinoline-1-spiro-4'-piperidine)-4-on** (alias: 15-[(6-chloro-3-pyridyl)methyl]spiro[1,2,3-trihydrobenzo [f]isoquinoline-1,4'-piperidine]-4-on (Japanese Patent Application No. 11-136812)); and

**1'-benzyl-7-methoxy(benzo[g]1,3-dihydroisobenzofuran-1-spiro-4'-piperidine)-3-imine** (alias: 7-methoxy-14-benzylspiro[hydrobenzo[e]isobenzofuran-1,4'-piperidine]-3-imine (Japanese Patent Application No. 11-136812)).

Novel compounds of the present invention shown in the above general formula (I) can be produced by the following method.

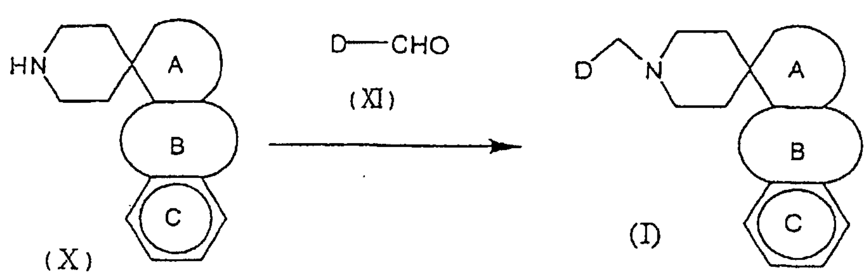


The compound of the above general formula (X) can be obtained by the following process: A base (preferably, lithium 2,2,6,6-tetramethylpyridine) is added to 2-naphthonitrile in a solvent which is not associated with the reaction (e.g. tetrahydrofuran, dimethoxyethane), and then 4-benzyloxycarbonylpiperidone is further added thereto. After the mixture is reacted at 0 to -80°C, preferably about -78°C, for 1 to 5 hours, preferably 2 hours, palladium black in formic acid is added. Then, the obtained mixture is reacted for 2 to 48 hours, preferably about 24 hours.

The compound of the above formula (XI) can be obtained as a commercially available reagent or obtained by the method described later in Reference examples 1 and 2 (*J. Med. Chem*. 37, 2729 (1994)); that is, an ester such as substituted methyl benzoate, substituted ethyl benzoate, substituted methyl nicotinate and substituted ethyl nicotinate, which are commercially available compounds, is reacted with a reducing agent such as lithium aluminum hydride at -10 to 50°C, preferably about 0 to 30°C, for 1 to 48 hours, preferably about 2 hours, in a solvent which is not associated with the reaction (e.g. tetrahydrofuran), followed by Swern oxidation or oxidation with manganese dioxide.

The compound of the above formula (I) can be obtained by reacting the compound of the above general formula (X) (in the formula (X), rings A, B and C have the same definitions as in the above general formula (I)) and the compound of the above general formula (XI) (in the formula (XI), D has the same definition as in the above general formula (I)) with sodium triacetoxy borohydride at 20 to 50°C, preferably about 30°C, for 2 to 48 hours, preferably about 5 hours, in a solvent which is not associated with the reaction (e.g. dichloroethane, tetrahydrofuran, dimethylsulfoxide), in the presence of acetic acid.

For the synthesis of the compound of general formula (I), the purification of the target compound from a reaction mixture can be performed by a method well used in synthetic chemistry, e.g. by a method of partitioning and extracting a reactant into water and an organic solvent inmiscible with water such as benzene, toluene, ethyl acetate, butyl acetate, methyl isobutylketone, chloroform and dichloromethane, followed by concentration, crystallization and so on. Moreover, fractional purification by a column chromatography with alumina or silica gel can be performed, as required.

The compounds of general formula (I) can be used in the form of pharmacologically acceptable salts thereof. Examples of possible salt forms include: acid addition salts with inorganic acids such as hydrochloric acid, nitric acid, hydrobromic acid and sulfuric acid; those with an aliphatic monocarboxylic acid, dicarboxylic acid, hydroxyalkanoic acid, hydroxyalkanoic diacid or amino acid; those induced from atoxic organic acids such as an aromatic acid, an aliphatic or aromatic sulfonic acid. Examples of such acid addition salts include hydrochloride, hydrobromate, nitrate, sulfate, hydrogensulfate, monohydrogenphosphate, dihydrogenphosphate, acetate, propionate, tartrate, oxalate, malonate, succinate, fumarate, maleate, mandelate, benzoate, phthalate, methanesulfonate, benzenesulfonate, toluenesulfonate, citrate, lactate, malate, glycolate and the like.

It became clear that, not only the compounds of the above general formula (I), but also any compound having both μ opioid receptor agonist activity and dopamine D2 receptor antagonist activity shows a potent analgetic effect and regulates side effects (in particular, regarding dopamine related behaviors). Therefore, according to the present invention, a compound having both μ opioid receptor agonist activity and dopamine D2 receptor antagonist activity (including the above new compound and a pharmacologically acceptable salt thereof) can be used as a new pain control agent with regulated side effects, that is, a useful pain control agent with a single agent.

Apart from the compound of the above general formula (I) of the present invention, any compound having both μ opioid receptor agonist activity and dopamine D2 receptor antagonist activity can be also used, and the examples include a compound shown in following general formula (II) or (III):

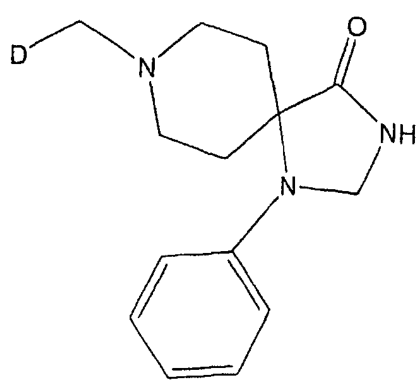

(II)

wherein D represents an aromatic ring, which may be substituted and may comprise a heteroatom(s) selected from S, N and O; and

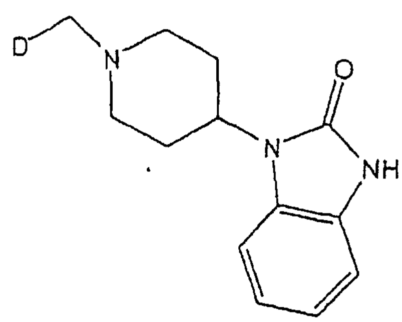

(III)

wherein D represents an aromatic ring, which may be substituted and may comprise a heteroatom(s) selected from S, N and O.

A group of compounds included in compounds of general formula (II) particularly preferable in the present invention includes a compound shown in the following general formula (VI) or pharmacologically acceptable salt thereof:

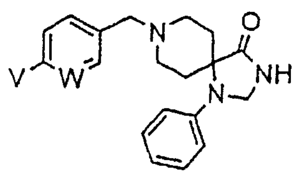

(VI)

wherein V is hydrogen, chlorine, bromine, methyl or methoxy; and W is nitrogen or CH.

In addition, another group of compounds included in compounds of general formula (II) particularly preferable in the present invention includes a compound shown in the following general formula (VII) or pharmacologically acceptable salt thereof:

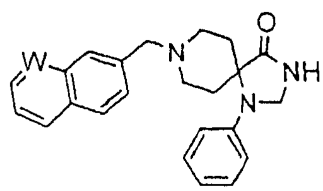

(VII)

wherein W is nitrogen or CH.

Furthermore, a group of compounds comprised in compounds of general formula (III) particularly preferable in the present invention includes a compound shown in the following general formula (VIII) or pharmacologically acceptable salt thereof:

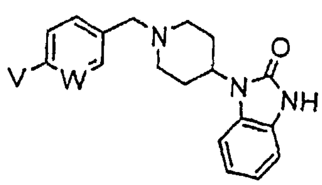

(VIII)

wherein V is chlorine or methoxy; and W is nitrogen or CH.

Compounds of general formulas (II) and (III) are described further in detail.

Just as with the above, the nomenclature used herein for compounds of general formulas (II) and (III) is described as follows. That is to say, throughout this specification, the compound shown in the following structural formula is referred as 1'-benzyl-spiroimidazolidine-4,4'-piperidine-3-phenyl-5-on.

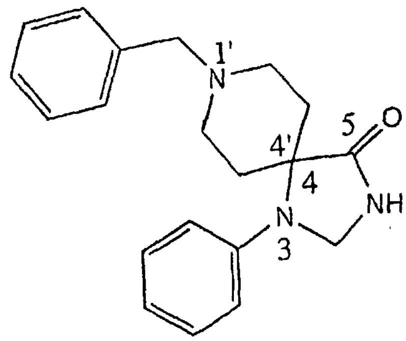

In Japanese Patent Application No. 11-136812, which is a priority application of the present application, the spirohydrocarbon number, heterocyclic ring atom number and the ring number characteristic of this structure are shown as follows, and the compound is referred as 1 -phenyl-8-benzyl-1,3,8-triazaspiro[4,5]decane-4-on.

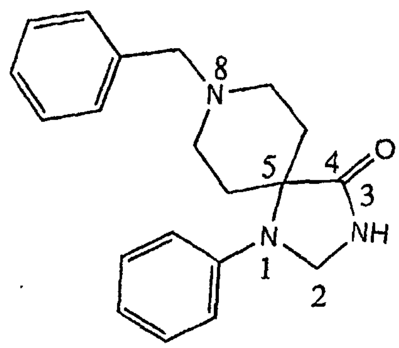

In general formulas (II) and (III), D adopts the same definition as in the above general formula (I).

Specific examples of a preferred group of compounds of general formula (II) and (III) include,

**1'-(4-methoxybenzyl)-spiroimidazolidine-4,4'-piperidine-3-phenyl-5-on** (alias: 1-phenyl-8-(4-methoxybenzyl)-1,3,8-triazaspiro[4,5]decane-4-on (Japanese Patent Application No. 11-136812));

**1'-(6-chloro-3-pyridyl)methyl-spiroimidazolidine-4,4'-piperidine-3-phenyl-5-on** (alias: 1-phenyl-8-[(6-chloro-3-pyridyl)methyl]-1,3,8-triazaspiro[4,5]decane-4-on (Japanese Patent Application No. 11-136812));

**1'-(6-methyl-3-pyridyl)methyl-spiroimidazolidine-4,4'-piperidine-3-phenyl-5-on** (alias: 1-phenyl-8-[(6-methyl-3-pyridyl)methyl]-1,3,8-triazaspiro[4,5]decane-4-on (Japanese Patent Application No. 11-136812));

**1'-(3-pyridyl)methyl-spiroimidazolidine-4,4'-piperidine-3-phenyl-5-on** (alias: 1-phenyl-8-(3-pyridyl)methyl-1,3,8-triazaspiro[4,5]decane-4-on (Japanese Patent Application No. 11-136812));

**1'-(4-bromobenzyl)-spiroimidazolidine-4,4'-piperidine-3-phenyl-5-on** (alias: 1-phenyl-8-(4-bromobenzyl)-1,3,8-triazaspiro[4,5]decane-4-on (Japanese Patent Application No. 11-136812));

**1-(4-methoxybenzyl)-4-(2-keto-1-benzoimidazolinyl)-piperidine;**

**1-[(6-chloro-3-pyridyl)methyl]-4-(2-keto-1-benzoimidazolinyl)-piperidine;**

**1'-(7-quinolylmethyl)-spiroimidazolidine-4,4'-piperidine-3-phenyl-5-on** (alias: 1-phenyl-8-(7-quinolylmethyl)-1,3,8-triazaspiro[4,5]decane-4-on (Japanese Patent Application No. 11-136812));

**1'-(2-naphtylmethyl)-spiroimidazolidine-4,4'-piperidine-3-phenyl-5-on** (alias: 1-phenyl-8-(2-naphtylmethyl)-1,3,8-triazaspiro[4,5]decane-4-on (Japanese Patent Application No. 11-136812)); and

**1'-(4-methylbenzyl)-spiroimidazolidine-4,4'-piperidine-3-phenyl-5-on** (alias: 1-phenyl-8-(4-methylbenzyl)-1,3,8-triazaspiro[4,5]decane-4-on (Japanese Patent Application No. 11-136812)).

Just as with the compounds of general formula (I), the compounds of general formulas (II) and (III) can be used in the form of pharmacologically acceptable salts thereof. Examples of possible salt forms include: acid addition salts with inorganic acids such as hydrochloric acid, nitric acid, hydrobromic acid and sulfuric acid; those with an aliphatic monocarboxylic acid, dicarboxylic acid, hydroxyalkanoic acid, hydroxyalkanoic diacid or amino acid; those induced from atoxic organic acids such as an aromatic acid, an aliphatic or aromatic sulfonic acid. Examples of such acid addition salts include hydrochloride, hydrobromide, nitrate, sulfate, hydrogensulfate, monohydrogenphosphate, dihydrogenphosphate, acetate, propionate, tartrate, oxalate, malonate, succinate, fumarate, maleate, mandelate, benzoate, phthalate, methanesulfonate, benzenesulfonate, toluenesulfonate, citrate, lactate, malate, glycolate and the like.
The compound of general formula (II) or (III) can be produced by the following method:

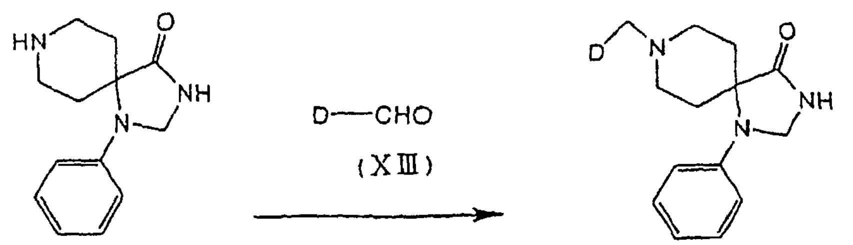


(XII) (II)

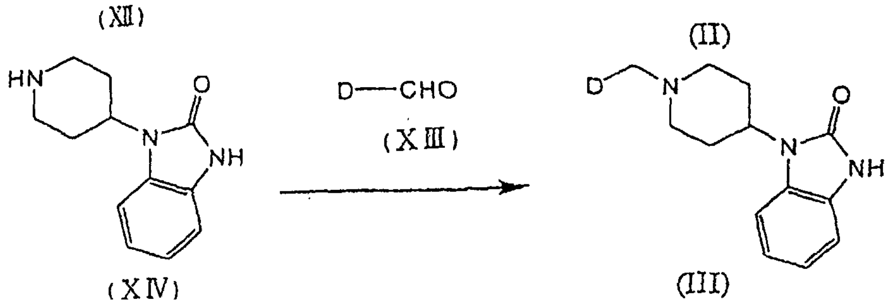


(XIV) (III)

Namely, the compounds shown in the above general formulas (XII) and (XIV) and the compound shown in the above general formula (XIII) (wherein D has the same definition as in the above general formulas (I) to (III)) are reacted at 20 to 50°C, preferably at about 30° C, for 2 to 48 hours, preferably about 5 hours, in a solvent which is not associated with the reaction (e.g, dichloroethane, tetrahydrofuran, dimethylsulfoxide), in the presence of sodium triacetoxy borohydride and acetic acid.
In addition, the compounds of the above formulas (XII) to (XIV) can be obtained as commercially available reagents. Furthermore, the compound of general formula (XIII) can be synthesized by the method described later in Reference examples 1 and 2. That is, an ester such as substituted methyl benzoate, substituted ethyl benzoate, substituted methyl nicotinate and substituted ethyl nicotinate, which are commercially available compounds, is reacted with a reducing agent such as lithium aluminum hydride at -10 to 50°C, preferably about 0 to 30°C, for 1 to 48 hours, preferably about 2 hours, in a solvent which is not associated with the reaction (e.g. tetrahydrofuran), followed by Swern oxidation or oxidation with manganese dioxide.
A pharmaceutical composition having, as an active ingredient, the compound of the present invention can be administered to both humans and non-human animals, through any route of administration such as oral and parenteral administration (e.g. intravenous injection, intramuscular injection, and subcutaneous, intrarectal, percutaneous and intraspinal administration). Therefore, the pharmaceutical composition having, as an active ingradient, the compound of the present invention adopts a suitable dosage form depending on the administration route.
Specifically, the oral dosage form includes tablets, capsules, powder, granules, syrup and so on, while the parenteral dosage form includes intravenous and intramuscular injections, agents for intrarectal administration, oleaginous suppositories and aqueous suppositories.
These various formulations can be produced according to standard techniques, with a commonly used excipient, disintegrator, binder, lubricant, colorant and so on.
Examples of excipients include lactose, glucose, corn starch, sorbit, crystalline cellulose etc., examples of disintegrators include starch, sodium alginate, gelatin powder, calcium carbonate, calcium citrate, dextrin etc., examples of binders include dimethylcellulose, polyvinyl alcohol, polyvinyl ether, methylcellulose, ethylcellulose, gum arabic,

gelatin, hydroxypropylcellulose, polyvinylpyrrolidone etc., and examples of lubricants include talc, magnesium stearate, polyethylene glycol, hydrogenated plant oil etc. Moreover, the above formulations can be produced, as needed, with the addition of buffer, pH regulator, stabilizer and the like.

The amount of the compound of the present invention contained in a pharmaceutical composition depends on its dosage form, but it is usually 0.1 to 50 weight %, preferably 0.5 to 20 weight % of the entire composition. The dosage may be determined as the case may be, considering, for example, age, body weight, sex, disease type and symptom of the patient, but it is usually 1 to 1,000mg, preferably 1 to 300mg per adult per day, and such dosage may be applied once or divided over several administrations per day.

## EXAMPLES

The present invention is further described in the following examples. The example is provided for illustrative purposes only, and is not intended to limit the scope of the invention.

### Reference example 1. 6-chloropyridine-3-carboaldehyde

0.20g (1.17mmol) of methyl 6-chloronicotinate (Lancaster) was dissolved in 4ml of tetrahydrofuran anhydrous, and 0.045g (1.17mmol) of lithium aluminum hydride was added thereto under ice bath cooling, followed by stirring for 30 minutes. Sodium sulfate 10 hydrate was added to the reaction mixture until no foam appeared, and after stirring for 2 hours, filtration with Celite was performed. The filtrate was evaporated, and the residue was purified with silica gel column chromatography (ethyl acetate) to obtain 0.090g of 6-chloropyridine-3-methyl alcohol.
$^1$H NMR ($CDCl_3$, 300MHz) $\delta$ 3.79(1H, brs), 4.70 (2H, s), 7.31 (1H, d, J = 8.3Hz), 7.69
(1H, dd, J = 2.4, 8.3Hz), 8.28 (1H,d, J= 2.4Hz)
mass spectrum EIMS, m/z :143($M^+$)

Subsequently, 1.5ml of methylene chloride anhydrous, in which 0.15ml (1.89mmol) of dimethylsulfoxide was dissolved, was added at -78°C to the reaction mixture, to which 2.8ml of methylene chloride anhydrous and 0.09ml (0.94mmol) of oxalylchloride were added, followed by stirring for 15 minutes. After that, 0.5ml of methylene chloride anhydrous, in which 0.09g (0.63mmol) of the above 6-chloropyridine-3-methyl alcohol was dissolved, was added and stirred for 1 hour. Then, 0.5ml (3.15mmol) of triethylamine was further added thereto, stirred for 1 hour and the temperature was raised to 0°C. Extraction was performed by adding 20ml of chloroform and 15ml of saturated sodium chloride solution thereto under ice bath cooling. After the organic layer was dried with magnesium sulfate anhydrous and the solvent was evaporated, the residue was purified with silica gel column chromatography (hexane : ethyl acetate = 1 : 1) to obtain 0.075g of the above compound of interest.
$^1$H NMR ($CDCl_3$, 300MHz) $\delta$ 7.53 (1H, d, J=8.3Hz), 8.16(1H, dd, J=2.4, 8.3Hz), 8.88 (1H,d, J=2.4Hz), 10.1 (1H, s)
mass spectrum EIMS, m/z : 141 ($M^+$)

### Reference example 2. 6-methylpyridine-3-carboaldehyde

1.00g (6.62mmol) of methyl 6-methyl nicotinate (Lancaster) was dissolved in 20ml of tetrahydrofuran anhydrous, and then 0.25g (6.59mmol) of lithium aluminum hydride was added thereto under ice bath cooling, followed by stirring for 30 minutes. Sodium sulfate 10 hydrate was added to the reaction mixture until no foam appeared, and after stirring for 2 hours, filtration with Celite was performed. The filtrate was subjected to vacuum concentration, and the residue was purified with silica gel column chromatography (ethyl acetate) to obtain 0.80g of 6-methylpyridine-3-methyl alcohol.

Subsequently, 4.3ml of methylene chloride anhydrous, in which 0.43ml (6.06mmol) of dimethylsulfoxide was dissolved, was added at -78°C to the reaction mixture, to which 8,0ml of methylene chloride anhydrous and 0.27ml (3.10mmol) of oxalylchloride were added, followed by stirring for 15 minutes. After that, 2.5ml of methylene chloride anhydrous, in which 0.25g (2.03mmol) of the above 6-methylpyridine-3-methyl alcohol was dissolved, was further added thereto, followed by stirring for 1 hour. 1.4ml (10.0mmol) of triethylamine was further added thereto, stirred for 1 hour and the temperature was raised to 0° C. Extraction was performed by adding 20ml of chloroform and 15ml of saturated sodium chloride solution thereto under ice bath cooling. After the organic layer was dried with magnesium sulfate anhydrous and the solvent was evaporated, the residue was purified with silica gel column chromatography (hexane : ethyl acetate = 1 : 1) to obtain 0.125g of the above compound of interest.
$^1$H NMR ($CDCl_3$, 300MHz) $\delta$ 2.67 (3H, s), 7.33 (1H, d, J=8.0H z ), 8.07 (1H, dd, J=2.0, 8.0Hz), 8.96 (1H, d, J=2.0Hz), 10.1 (1H, s)
mass spectrum EIMS, m/z : 121 ($M^+$)

**Reference example 3.**

1'-benzyloxycarbonyl(benzo[g]1,3-dihydroisobenzofuran-1-spiro-4'-piperidine)-3-imine

1'-benzyloxycarbonyl(benzo[f]1,3-dihydroisobenzofuran-1-spiro-4'-piperidine)-3-imine

0.85g (6.02mmol) of 2,2,6,6-tetramethylpiperidine was dissolved in 25ml of tetrahydrofuran anhydrous. The reaction mixture was set to 0°C, and 5.3ml of methyl lithium diethyl ether solution (1.14mol/l) was added thereto, followed by stirring for 30 minutes. The temperature of the reaction mixture was reduced to -78°C, and 0.92g of 2-naphthonitrile dissolved in 3ml of tetrahydrofuran anhydrous was dropped in the reaction mixture, followed by stirring for 50 minutes. Subsequently, 1.54g (6.60mmol) of 4-benzyloxycarbonylpiperidone dissolved in 5ml of tetrahydrofuran anhydrous was added thereto, followed by stirring for 2 hours. The temperature was raised to 0°C followed by stirring for 1 hour, and then further stirring for 1 hour at room temperature. Extraction was performed by adding 50ml of methylene chloride, 10ml of water and 5ml of 2mol/l hydrochloric acid solution to the reaction mixture at 0°C. After the organic layer was dried with magnesium sulfate anhydrous and the solvent was evaporated, the residue was purified with silica gel column chromatography (methylene chloride : methanol = 30 : 1) to obtain 0.10g of 1'-benzyloxycarbonyl (benzo[g]1,3-dihydroisobenzofuran-1-spiro-4'-piperidine)-3-imine and 0.07g of 1'-benzyloxycarbonyl(benzo[f] 1,3-dihydroisobenzofuran-1-spiro-4'-piperidine)-3-imine.

1'-benzyloxycarbonyl(benzo[g]1,3-dihydroisobenzofuran-1-spiro-4'-piperidine)-3-imine

$^1$H NMR ($CDCl_3$, 300MHz) δ 1.76(2H, brd), 2.50-2.64 (2H, m), 3.34-3.52 (2H, m), 4.22-4.44 (2H, m), 5.24 (2H, s), 7.21-8.15 (11 H, m)
mass spectrum EIMS, m/z : 386($M^+$)

1'-benzyloxycarbonyl(benzo[f]1,3-dihydroisobenzofuran-1-spiro-4'-piperidine)-3-imine

$^1$H NMR($CDCl_3$, 300MHz) δ 1.65-1.95 (2H, m), 2.00-2.70 (2H, m), 3.10-3.55 (2H, m), 3.90-4.50 (2H,m), 5.21 (2H,s), 7.23-8.50 (11H, m)
mass spectrum EIMS, m/z : 386 ($M^+$)

**Reference example 4.**

benzo[g]1,3-dihydroisobenzofuran-1-spiro-4'-piperidine-3-imine

0.095g (0.250mmol) of the compound obtained in Reference example 3. (1'-benzyloxycarbonyl(benzo[g] 1,3-dihydroisobenzofuran-1-spiro-4'-piperidine)-3-imine), was dissolved in 1.4ml of formic acid, and 0.057g of palladium black was added thereto, followed by stirring for 24 hours in an argon atmosphere. Then, 0.03g of palladium black was further added and stirred for 24 hours again. The reaction solution was filtrated with Celite, and the obtained filtrate was evaporated. Extraction was performed by adding 10ml of chloroform, 10ml of water and 20ml of saturated sodium bicarbonate solution to the residue. After the organic layer was dried with magnesium sulfate anhydrous and the solvent was evaporated to obtain 0.02g of the above compound of interest.
$^1$H NMR ($CDCl_3$, 300MHz) δ 1.78(2H, d, J=13.0Hz), 2.62(2H, dt, J=5.2, 13.0Hz), 3.12-3.24 (2H, m), 3.26 (2H, d t, J=2.5, 13.0Hz), 7.58-7.66 (2H, m), 7.81-7.88 (1H, m), 7.91-8.03 (2H, m), 8.17-8.23 (1H, m)
mass spectrum EIMS, m/z : 252($M^+$)

**Reference example 5.**

benzo[f]1,3-dihydroisobenzofuran-1-spiro-4'-piperidine-3-imine

0.072g (0.186mmol) of the compound obtained in Reference example 3. (1'-benzyloxycarbonyl(benzo[f] 1,3-dihydroisobenzofuran-1-spiro-4'-piperidine)-3-imine), was dissolved in 1.4ml of formic acid, and 0.043g of palladium black was added thereto, followed by stirring for 24 hours in an argon atmosphere. Then, 0.03g of palladium black was further added and stirred for 24 hours again. The reaction solution was filtrated with Celite, and the obtained filtrate was evaporated. Extraction was performed by adding 10ml of chloroform, 10ml of water and 20ml of saturated sodium bicarbonate solution to the residue. After the organic layer was dried with magnesium sulfate anhydrous and the solvent was evaporated to obtain 0.017g of the above compound of interest.
$^1$H NMR ($CDCl_3$, 400MHz) δ 1.70-1.90 (2H,m), 2.22 (2H, dt, J=5.2, 13.7Hz), 3.12-3.30 (4H, m), 7.60 (1H, ddd, J=1.4,

6.8, 7.8Hz), 7.67 (1H, ddd, J=1.4, 6.8, 7.8Hz), 7.83 (1H, s), 7.96 (1H, d, J=7.8Hz), 8.05 (1H, d, J=7.8Hz), 8.47 (1H, s)
mass spectrum EIMS, m/z : 252($M^+$)

**Example 1. 1'-(6-chloro-3-pyridyl)methyl(benzo[g]1,3-dihydroisobenzofuran-1-spiro-4'-piperidine)-3-imine**

(alias: 1'-(2-chloropyridyl)-benzo[c]spiro[benzodihydrofuran-1(3H),4'-piperidine]-3-imine, or 14-[(6-chloro-3-pyridyl) methyl]spiro[hydrobenzo[e]isobenzofuran-1,4'-piperidine]-3-imine)

0.042 g (0.166mmol) of the compound obtained in Reference example 4. and 0.024g (0.169mmol) of 6-chloropyridine-3-carboaldehyde obtained in Reference example 1. were dissolved in 0.84ml of 1,2-dichloroethane, and then 0.09ml (1.66mmol) of acetic acid and 0.053g (0.25mmol) of sodium triacetoxy borohydride were added thereto, followed by stirring for 12 hours. Extraction was performed by adding 10ml of chloroform, 5ml of water and 5ml of saturated sodium bicarbonate solution to the reaction solution. After the organic layer was dried with magnesium sulfate anhydrous and the solvent was evaporated, the residue was purified with silica gel column chromatography (methylene chloride : methanol = 20 : 1) to obtain 0.020g of the above compound of interest.
$^1$H NMR ($CDCl_3$, 300MHz) δ 1.80(2H, brd), 2.59-2.85 (4H, m), 2.85-3.00 (2H, m), 3.65 (2H, s), 7.34 (1H, d, J=8.3Hz), 7.60-7.80 (3H, m), 7.84 (1H, t, J=8.3Hz), 7.90-8.08 (2H, m), 8.12 (1H, d, J=7.2Hz), 8.44 (1H, d, J=2.4Hz)
mass spectrum EIMS, m/z: 377($M^+$)

**Example 2.**

**1'-(6-chloro-3-pyridyl)methyl(benzo[g]1,3-dihydroisobenzofuran-1-spiro-4'-piperidine)-3-on**

(alias: 1'-(2-chloropyridyl)-benzo[c]spiro[benzodihydrofuran-1(3H),4'-piperidine]-3-on, or 14-[(6-chloro-3-pyridyl) methyl]spiro[hydrobenzo[e]isobenzofuran-1,4'-piperidine]-3-on)

0.012g (0.032mmol) of the compound obtained in example 1. was dissolved in 0.25ml of methylene chloride, and 0.03g (0.127mmol) of camphor sulfonic acid was added thereto, followed by stirring. At each of 2, 20 and 44 hours after initiation of the reaction, 0.03g (0.127mmol) of camphor sulfonic acid was further added, and at 28 hours after initiation of the reaction, 0.1ml of water was further added. At 66 hours after initiation of the reaction, extraction was performed by adding 3ml of chloroform and 2ml of saturated sodium bicarbonate solution. After the organic layer was dried with magnesium sulfate anhydrous and the solvent was evaporated, the residue was purified with silica gel column chromatography (methylene chloride : methanol = 20 : 1) to obtain 0.006g of the above compound of interest.
$^1$H NMR ($CDCl_3$, 300MHz) δ 1.80(2H, brd), 2.67-2.85 (4H, m), 2.95(2H, d, J=5.9Hz), 3.66 (2H, s), 7.34 (1H, dd, J=1.3, 8.3Hz), 7.69-7.77 (3H, m), 7.80 (1H, dd, J=1.3, 8.3Hz), 7.98 (1H, d, J=8.3Hz), 8.02-8.08 (1H, m), 8.15-8.23 (1H, m), 8.46(1H, s)
mass spectrum EIMS, m/z: 378($M^+$)

**Example 3.**

**1'-(6-chloro-3-pyridyl)methyl(benzo[f]1,3-dihydroisobenzofuran-1-spiro-4'-piperidine)-3-on**

(alias: 1'-(2-chloropyridyl)benzo[d]spiro[benzodihydrofuran-1(3H),4'-piperidine]-3-on, or 14-[(6-chloro-3-pyridyl) methyl]spiro[hydrobenzo[f]isobenzofuran-3,4'-piperidine]-1-on)

0.020 g (0.079mmol) of the compound obtained in Reference example 5. and 0.011g (0.077mmol) of 6-chloropyridine-3-carboaldehyde obtained in Reference example 1. were dissolved in 0.40ml of 1,2-dichloroethane, and then 0.044ml (0.79mmol) of acetic acid and 0.025g (0.12mmol) of sodium triacetoxy borohydride were added thereto, followed by stirring for 12 hours. Extraction was performed by adding 10ml of chloroform, 5ml of water and 5ml of saturated sodium bicarbonate solution to the reaction solution. After the organic layer was dried with magnesium sulfate anhydrous and the solvent was evaporated, the residue was purified with silica gel column chromatography (methylene chloride : methanol = 20 : 1) to obtain 0.010g of the above compound of interest.
$^1$H NMR ($CDCl_3$, 400MHz) δ 1.84(2H, brd), 2.32(2H, d, J=4.6, 13.0Hz), 2.64 (2H, d, J=2.2, 13.0Hz), 2.91 (2H, brd), 3.63 (2H, s), 7.33 (1H, d, J=8.0Hz), 7.60 (1H, ddd, J=1.2, 7.8, 8.3Hz), 7.67 (1H, ddd, J=1.2, 8.0, 8.3Hz), 7.71 (1H, dd, J=2.2, 8.0Hz), 7.84(1H, s), 7.96 (1H, d, J=8.0Hz), 8.04 (1H, d, J=7.8Hz), 8.41 (1H, d, J=2.2Hz), 8.46 (1H, s)
mass spectrum EIMS, m/z: 378($M^+$)

**Example 4.**

**1'-(4-fluorophenyl)methyl(benzo[g]1,3-dihydroisobenzofuran-1-spiro-4'-piperidine)-3-imine**

(alias: 1'-(4-fluorobenzyl)-benzo[c]spiro[benzodihydrofuran-1(3H),4'-piperidine]-3-imine, or 14-(4-fluorobenzyl)spiro [hydrobenzo[e]isobenzofuran-1,4'-piperidine]-3-imine)

12.6mg (50 μ mol) of the compound obtained in Reference example 4. and 11 μ 1 (100 μ mol) of 4-fluorobenzaldehyde (Tokyo Kasei Kogyo Co., Ltd.) were dissolved in 2ml of 1,2-dimethoxyethane (1% acetic acid), and then 43.4mg (205 μ mol) of sodium triacetoxy borohydride was added thereto, followed by stirring at room temperature for 2 hours. The reaction solution was evaporated, and the residue was purified with silica gel column chromatography (ethyl acetate : hexane = 1 : 2 to methylene chloride : methanol = 8 : 1) to obtain 17.4mg of the above compound of interest.
$^1$H NMR ($CDCl_3$, 400MHz) δ 1.83 (2H, d, J=12.7 Hz), 2.80-2.98 (4H, m), 3.25 (2H, brd), 3.89 (2H, s), 7.09 (2H, t, J=8.5Hz), 7.43 (2H, dd, J=5.3, 8.5Hz), 7.62-7.67 (2H, m), 7.84 (1H, d, J=8.5Hz), 7.94-8.01 (2H, m), 8.21 (1H, dd, J=4.2, 5.3Hz)
mass spectrum TSPMS, m/z :361$(M+H)^+$

**Example 5.**

**1'-(4-chlorophenyl)methyl(benzo[g]1,3-dihydroisobenzofuran-1-spiro-4'-piperidine)-3-imine**

(alias: 1'-(4-chlorobenzyl)-benzo[c]spiro[benzodihydrofuran-1(3H),4'-piperidine]-3-imine, or 14-(4-chlorobenzyl)spiro [hydrobenzo[e]isobenzofuran-1,4'-piperidine]-3-imine)

Using 12.6mg (50 μ mol) of the compound obtained in Reference example 4. and 14mg (100 μ mol) of 4-chlorobenzaldehyde (Nacalai Tesque, Inc.), 15.9mg of the above compound of interest was obtained by the same method as shown in Example 4.
$^1$H NMR ($CDCl_3$, 400MH z ) δ 1.82(2H, d, J=12.4Hz), 2.75-2.92 (4H, m), 3.18(2H, brd), 3.84(2H, s), 7.31-7.40 (4H, m), 7.62-7.67 (2H, m), 7.84 (1H, d, J=8.5Hz), 7.93 - 8.01 (2H, m), 8.20 (1H. dd, J=2.9, 9.5Hz)
mass spectrum TSPMS, m/z: 377$(M+H)^+$

**Example 6.**

**1'-(4-methoxyphenyl)methyl(benzo[g]1,3-dihydroisobenzofuran-1-spiro-4'-piperidine)-3-imine**

(alias: 1 '-(4-methoxybenzyl)-benzo[c]spiro[benzodihydrofuran-1(3H),4'-piperidine]-3-imine, or 14-(4-methoxybenzyl) spiro[hydrobenzo[e]isobenzofuran-1,4'-piperidine]-3-imine)

Using 12.6mg (50 μ mol) of the compound obtained in Reference example 4. and 14mg (100 μ mol) of 4-methoxybenzaldehyde (Tokyo Kasei Kogyo Co., Ltd.), 18.9mg of the above compound of interest was obtained by the same method as in Example 4.
$^1$H NMR ($CDCl_3$, 400MH z ) δ 1.83(2H, d, J=13.4Hz), 2.82-3.02 (4H, m), 3.29(2H, brd), 3.83(3H, s), 3.90 (2H, s), 6.91-6.95 (2H, m), 7.37 (2H, d, J=8.5Hz), 7.61-7.67 (2H, m), 7.82 (1H, d, J=8.5Hz), 7.93-8.00 (2H, m), 8.24 (1H, dd, J=2.4, 9.0Hz)
mass spectrum TSPMS, m/z: 373$(M+H)^+$

**Example 7.**

**1'-(4-fluorophenyl)methyl(benzo[g]1,3-dihydroisobenzofuran-1-spiro-4'-piperidine)-3-on**

(alias: 1'-(4-fluorobenzyl)-benzo[c]spiro[benzodihydrofuran-1(3H),4'-piperidine]-3-on, or 14-(4-fluorobenzyl)spiro [hydrobenzo[e]isobenzofuran-1,4'-piperidine]-3-on)

17.4mg of the compound obtained in Example 4 was dissolved in 0.75ml of chloroform and left at room temperature overnight to obtain 17.4mg of the above compound of interest.
$^1$H NMR ($CDCl_3$, 400MH z ) δ 1.72 (2H, brd), 2.61-2.80 (4H, m), 2.99(2H, brd), 3.64 (2H, s), 6.96-7.01 (2H, m), 7.29-7.34 (2H, m), 7.54-7.64 (2H, m), 7.79 (1H, m), 7.88-7.97 (2H, m), 8.14 (1H, m)

mass spectrum TSPMS, m/z: 362$(M+H)^+$

**Example 8.**

**1'-(4-chlorophenyl)methyl(benzo[g]1,3-dihydroisobenzofuran-1-spiro-4'-piperidine)-3-on**

(alias: 1'-(4-chlorobenzyl)-benzo[c]spiro[benzodihydrofuran-1(3H),4'-piperidine]-3-on, or 14-(4-chlorobenzyl)spiro[hydrobenzo[e]isobenzofuran-1,4'-piperidine]-3-on)

15.9mg of the compound obtained in Example 5 was dissolved in 0.75ml of chloroform and left at room temperature overnight to obtain 15.9mg of the above compound of interest.
$^1$H NMR ($CDCl_3$, 400MH z ) δ 1.77-1.81 (2H, brd), 2.63 - 2.85 (4H, m), 3.00-3.02 (2H, m), 3.68 (2H, s), 7.33-7.39 (4H, m), 7.62-7.71 (2H, m), 7.84 (1H, dd, J=5.6, 8.5Hz), 7.92-8.05 (2H, m), 8.20 (1H, m)
mass spectrum TSPMS, m/z: 378$(M+H)^+$

**Example 9.**

**1'-(4-methoxyphenyl)methyl(benzo[g]1,3-dihydroisobenzofuran-1-spiro-4'-piperidine)-3-on**

(alias: 1'-(4-methoxybenzyl)-benzo[c]spiro[benzodihydrofuran-1(3H),4'-piperidine]-3-on, *or* 14-(4-methoxybenzyl) spiro[hydrobenzo[e]isobenzofuran-1,4'-piperidine]-3-on)

18.9mg of the compound obtained in Example 6 was dissolved in 0.75ml of chloroform and left at room temperature overnight to obtain 18.9mg of the above compound of interest.
$^1$H NMR ($CDCl_3$, 400MH z ) δ 1.79 (2H, brd), 2.65-2.88 (4H, m), 3.07 (2H, brd), 3.70 (2H, s), 3.83 (3H, s), 6.91 (2H, d, J=7.3Hz), 7.29-7.36 (2H, m), 7.60-7.70 (2H, m), 7.83 (1H, dd, J=7.3, 8.3Hz), 7.92 - 8.04 (2H, m), 8.18 - 8.27 (1H, m)
mass spectrum TSPMS, m/z: 374$(M+H)^+$

**Example 10.**

**1'-benzyl(benzo[g]1,3-dihydroisobenzofuran-1-spiro-4'-piperidine)-3-imine**

(alias: 1'-benzyl-benzo[c]spiro[benzodihydrofuran-1(3H),4'-piperidine]-3-imine, or 14-benzylspiro[hydrobenzo[e] isobenzofuran-1,4'-piperidine]-3-imine)

0.85g (6.02mmol) of 2,2,6,6-tetramethylpiperidine was dissolved in 25ml of tetrahydrofuran anhydrous. The reaction mixture was set to 0°C, and 5.3ml of 1,14mol/l methyl lithium diethyl ether solution was added thereto, followed by stirring for 30 minutes. The temperature of the reaction mixture was reduced to -78°C, and 0.92g of 2-naphthonitrile dissolved in 3ml of tetrahydrofuran anhydrous was added dropwise to the reaction mixture, followed by stirring for 50 minutes. Subsequently, 1.25g (6.60mmol) of 4-benzylpiperidone dissolved in 5ml of tetrahydrofuran anhydrous was added thereto, followed by stirring for 2 hours. The temperature was raised to 0°C followed by stirring for 1 hour, and then further stirring at room temperature for 1 hour. Extraction was performed by adding 50ml of methylene chloride, 10ml of water and 5ml of 2mol/l hydrochloric acid solution to the reaction mixture at 0°C. After the organic layer was dried with magnesium sulfate anhydrous and the solvent was evaporated, the residue was purified with silica gel column chromatography (methylene chloride : methanol = 20 : 1) to obtain 0.46g of the above compound of interest.
$^1$H NMR ($CDCl_3$, 400MHz) δ 1.79(2H, brd), 2.59 (2H, brt), 2.74 (2H, dt, J=4.4, 13.0Hz), 2.97 (2H, dd, J=1.8, 8.3Hz), 3.68 (2H, s), 7.25-7.50 (5H, m), 7.55-7.70 (2H, m), 7.84 (1H, brs), 7.92 (1H, d, J =8.6Hz), 7.99 (1H, d, J=7.3Hz), 8.18 (1H, d, J=7.3 Hz)
mass spectrum EIMS, m/z: 342$(M^+)$

**Example 11.**

**1'-benzyl(benzo[g]1,3-dihydroisobenzofuran-1-spiro-4'-piperidine)-3-on**

(alias: 1'-benzyl-benzo[c]spiro[benzodihydrofuran-1(3H),4'-piperidine]-3-on, or 14-benzylspiro[hydrobenzo[e] isobenzofuran-1,4'-piperidine]-3-on)

0.015g (0.044mmol) of 1'-benzyl (benzo[g]1,3-dihydroisobenzofuran-1-spiro-4'-piperidine)-3-imine obtained

in Example 10 was dissolved in 0.15ml of methylene chloride, and then 0.01g (0.044mmol) of camphor sulfonic acid was added thereto, followed by stirring. At 18 hours after initiation of the reaction, 0.03g (0.131mmol) of camphor sulfonic acid was further added. At 22 hours after initiation of the reaction, extraction was performed by adding 3ml of chloroform and 2ml of saturated sodium bicarbonate solution. After the organic layer was dried with magnesium sulfate anhydrous and the solvent was evaporated, the residue was purified with silica gel column chromatography (methylene chloride : methanol = 20 : 1) to obtain 0.006g of the above compound of interest.
$^{1}$H NMR ($CDCl_3$, 300MHz) δ 1.78(2H, brd), 2.62-2.87 (4H, m), 3.00 (2H, brd), 3.69 (2H, s), 7.22-7.47 (5H, m), 7.65-7.75 (2H, m), 7.85 (1H, d, J=8.5Hz), 7.97 (1H, d, J=8.5Hz), 8.00-8.06 (1H, m), 8.21-8.28 (1H, m)
mass spectrum EIMS,m/z:343($M^+$)

**Reference example 6. bis(2-chloroethyl)benzylamine**

5.00g (47.6mmol) of diethanolamine (Tokyo Kasei Kogyo Co., Ltd.) was dissolved in 100ml of N,N-dimethylformamide anhydrous, and then 8.70g (62.9mmol) of potassium carbonate and 6.52ml (54.8mmol) of benzyl bromide were added thereto followed by stirring for 21 hours. Extraction was performed by adding 200ml of water and 200ml of methylene chloride to the reaction mixture. After the organic layer was dried with magnesium sulfate anhydrous and the solvent was evaporated, the residue was purified with silica gel column chromatography (methylene chloride : methanol = 10 : 1) to obtain 8.4g of bis(2-hydroxyethyl)benzylamine.

Then, 45ml of methylene chloride and 75ml of thionyl chloride were added to the obtained 8.4g (43.0mmol) of bis(2-hydroxyethyl)benzylamine and stirred for 12 hours. 100ml of methylene chloride and 500ml of saturated sodium bicarbonate solution were added to the reaction mixture at 0°C, and then this reaction solution was poured into a solution containing 250g of saturated sodium bicarbonate, followed by extraction with methylene chloride. After the organic layer was dried with magnesium sulfate anhydrous and the solvent was evaporated, the residue was purified with silica gel column chromatography (hexane : ethyl acetate = 4 : 1) to obtain 8.8g of the above compound of interest.
mass spectrum EIMS, m/z: 231, 233 ($M^+$)

**Reference example 7. 1 -benzyl-4-cyano-4-naphtylpiperidine**

6.40g (38.3mmol) of 1-naphthaleneacetonitrile (Tokyo Kasei Kogyo Co., Ltd.) was dissolved in 64ml of dimethylsulfoxide anhydrous, and then 2.01g (83.8mmol) of sodium hydride washed with n-hexane was added thereto, followed by stirring for 30 minutes. 8.90g (38.3mmol) of bis(2-chloroethyl)benzylamine obtained in Reference example 6. was added thereto, and stirred, first, at 75°C for 1 hour in an oil bath, then at room temperature for 1 hour. Extraction was performed by adding 300ml of water and 300ml of diethyl ether thereto under ice bath cooling. After the organic layer was dried with magnesium sulfate anhydrous and the solvent was evaporated, the residue was purified with silica gel column chromatography (hexane : ethyl acetate = 2 : 1) to obtain 10.7g of the above compound of interest.
$^{1}$H NMR ($CDCl_3$, 300MHz) δ 2.20(2H, brt), 2.60 (2H, brd), 2.74 (2H, dt, J=1.9Hz, J =12.3Hz), 3.07 (2H, brd), 3.65 (2H, s), 7.25-7.65 (9H, m), 7.85 (1H, d, J=7.7Hz), 7.91 (1H, d, J=8.0Hz), 8.53 (1H, d, J=8.0Hz)
mass spectrum EIMS, m/z: 326($M^+$)

**Reference example 8. 4-cyano-1-ethoxycarbonyl-4-naphtylpiperidine**

10.7g (32.8mmol) of the compound obtained in Reference example 7 was dissolved in 100ml of methylene chloride, and then 3.94g (39.4mmol) of potassium bicarbonate and 3.8ml (39.7mmol) of ethyl chlorocarbonate were added thereto, followed by stirring for 20 hours. Extraction was performed by adding 200ml of methylene chloride and 150ml of water. After the organic layer was dried with magnesium sulfate anhydrous and the solvent was evaporated, the residue was purified with silica gel column chromatography (hexane : acetone = 4 : 1) to obtain 9.8g of the above compound of interest.
$^{1}$H NMR (CDCl3, 300MHz) δ 1.29(3H, t, J=7.1Hz), 2.05 (2H, dt, J=4.2Hz, J=13.2Hz), 2.63 (2H, brd), 3.47 (2H, brt), 4.18 (2H, q, J=7.1Hz), 4.40 (2H, brs), 7.43-7.67 (4H, m), 7.84-7.91 (1H, m), 7.93 (1H, d, J=8.0Hz), 8.51 (1H, d, J=8.7Hz)
mass spectrum EIMS, m/z: 308($M^+$)

**Reference example 9. 4-aminomethyl-1-ethoxycarbonyl-4-naphtylpiperidine**

9.8g (31.8mmol) of the compound obtained in Reference example 8 was dissolved in 250ml of ethanol, then 5.0g of palladium carbon and 13.2ml (66mmol) of 5N hydrochloric acid were added thereto, followed by stirring for 10 hours in a hydrogen atmosphere. 5.0g of palladium carbon was further added and stirred for 24 hours. The reaction solution was filtrated with Celite, the filtrate was evaporated, and the residue was purified with silica gel column chromatography (hexane : acetone = 4 : 1) to obtain 9.8g of the above compound of interest.

mass spectrum EIMS, m/z: 312 ($M^+$)

**Reference example 10.** 4-ethoxycarbonylaminomethyl-1-ethoxycarbonyl-4-naphtylpiperidine

7.61g (24.4mmol) of the compound obtained in Reference example 9 was dissolved in 66ml of methylene chloride, then 2.5ml (26.0mmol) of ethyl chlorocarbonate and 3.5ml (25.1mmol) of triethylamine were added thereto, followed by stirring for 2 hours. Extraction was performed by adding 100ml of methylene chloride and 75ml of saturated sodium bicarbonate solution to the reaction mixture. After the organic layer was dried with magnesium sulfate anhydrous and the solvent was evaporated, the residue was purified with silica gel column chromatography (hexane : ethyl acetate = 2: 1) to obtain 7.97g of the above compound of interest.
$^1$H NMR ($CDCl_3$, 300MHz) δ 1.15(3H, t, J=7.1Hz), 1.24 (3H, t, J=7.1Hz), 2.05-2.21 (2H, m), 2.40-2.53 (2H, m), 3.37 (2H, brt), 3.67-3.79 (2H, m), 3.95-4.25 (6H, m), 7.43-7.55 (4H, m), 7.80 (1H, dt, J=4.7Hz), 7.89-7.94 (1H, m), 8.40 (1H, brd)
mass spectrum EIMS, m/z: 384($M^+$)

**Reference example 11.**

benzo[h]1,2,3-trihydroisoquinoline-1-spiro-4'-piperidine-4-on

79g of polyphosphoric acid was added to 7.97g (28.8mmol) of the compound obtained in Reference example 10, and stirred at 150°C for 1 hour in an oil bath. Extraction was performed by adding 300ml of ice water, 300ml of methylene chloride and 350ml of 5N sodium hydroxide solution to the reaction mixture. The organic layer was dried with magnesium sulfate anhydrous and the solvent was evaporated to obtain 2.27g of the above compound of interest.
$^1$H NMR ($CDCl_3$, 300MHz) δ 1.70 (2H, brd), 2.90-3.05 (4H, m), 3.15 (2H, brd), 3.72 (2H, brd), 7.09 (1H, brs), 7.50-7.60 (2H, m), 7.81 (1H, d, J=8.5Hz), 7.85-7.92 (1H, m), 8.17 (1H, d, J=8.5Hz), 8.73-8.81 (1H, m)
mass spectrum EIMS, m/z: 266($M^+$)

**Example 12.**

**1'-([(6-chloro-3-pyridyl)methyl](benzo[h]1,2,3-trihydroisoquinoline-1-spiro-4'-piperidine)-4-on**

0.02g (0.075mmol) of the compound obtained in Reference example 11 and 0.01g (0.077mmol) of 6-chloropyridine-3-carboaldehyde obtained in Reference example 1. were dissolved in 2.0ml of 1,2-dichloroethane, then 0.043ml (0.78mmol) of acetic acid and 0.025g (0.12mmol) of sodium triacetoxy borohydride were added thereto, followed by stirring for 12 hours. Extraction was performed by adding 3.0ml of chloroform, 1.0ml of water and 3.0ml of saturated sodium bicarbonate solution to the reaction solution. After the organic layer was dried with magnesium sulfate anhydrous and the solvent was evaporated, the residue was purified with silica gel column chromatography (methylene chloride : methanol = 20 : 1) to obtain 0.015g of the above compound of interest.
$^1$H NMR ($CDCl_3$, 300MHz) δ 1.87 (2H, brd), 2.31 (2H, dt, J=3.0Hz, J=12.6Hz), 2.91 (2H, dd, J=4.6Hz, J=12.6Hz), 3.05 (2H, dt, J=4.6Hz, J=12.6Hz), 3.60 (2H, s), 3.64 (2H, d, J=3.0Hz), 6.35 (1H, s), 7.36 (1H, d, J=8.3Hz), 7.52-7.61 (2H, m), 7.75 (1H, dt, J=2.3Hz, J=8.3Hz), 7.81 (1H, d, J=8.3Hz), 7.86-7.93 (1H, m), 8.16 (1H, d, J=8.3Hz), 8.45 (1H, d, J=2.3Hz), 8.65-8.73 (1H, m)
mass spectrum EIMS, m/z: 390, 392($M^+$)

**Example 13.**

**1'-benzyl-7-methoxy(benzo[g]1,3-dihydroisobenzofuran-1-spiro-4'-piperidine)-3-imine**

(alias: 7-methoxy-14-benzy]spire[hydrobenzo[e]isobenzofuran-1,4'-piperidine]-3-imine), and

**1'-benzyl-7-methoxy(benzo[f]1,3-dihydroisobenzofuran-1-spiro-4'-piperidine)-3-imine**

1.93g (13.6mmol) of 2,2,6,6-tetramethylpiperidine was dissolved in 48ml of tetrahydrofuran anhydrous. The reaction mixture was set to 0°C, and 12ml of 1.14mol/l methyl lithium diethyl ether solution was added thereto, followed by stirring for 30 minutes. The temperature of the reaction mixture was reduced to -78°C, and 0.92g of 2-naphthonitrile dissolved in 10ml of tetrahydrofuran anhydrous was dropped in the reaction mixture, followed by stirring for 50 minutes. Subsequently, 2.55g (13.5mmol) of 4-benzylpiperidone dissolved in 10ml of tetrahydrofuran anhydrous was added thereto, followed by stirring for 2 hours. The temperature was raised to 0°C, followed by stirring for 1 hour, and the

further stirring for 1 hour at room temperature. Extraction was performed by adding 50ml of methylene chloride, 10ml of water and 5ml of 2mol/l hydrochloric acid solution to the reaction mixture at 0°C. After the organic layer was dried with magnesium sulfate anhydrous and the solvent was evaporated, the residue was purified with silica gel column chromatography (methylene chloride : methanol = 30 : 1) to obtain 1.01g of 1'-benzyl-7-methoxy(benzo[g]1,3-dihydroisobenzofuran-1-spiro-4'-piperidine)-3-imine (hereinafter referred to compound (A)) and 0.25g of 1'-benzyl-7-methoxy (benzo[f]1,3-dihydroisobenzofuran-1-spiro-4'-piperidine)-3-imine (hereinafter referred to compound (B)).
1'-benzyl-7-methoxy(benzo[g]1,3-dihydroisobenzofuran-1-spiro-4'-piperidine)-3-imine $^1$H NMR (CDC $1_3$, 400MHz) δ 1.76 (2H,, brd), 2.52-2.75 (4H, m), 2.94 (2H, brd), 3.67 (2H, s), 3.95 (3H, s), 7.25-7.40 (7H, m), 7.76 (2H, brd), 8.07 (1H, brs)
mass spectrum EIMS, m/z: 372($M^+$)
1 '-benzyl-7-methoxy(benzo[f]1,3-dihydroisobenzofuran-1 -spiro-4'-piperidine)-3-imine $^1$H NMR ($CDCl_3$, 400MHz) δ 1.76(2H, brd), 2.22-3.04 (6H, m), 3.55-3.68 (2H, m), 3.95 (3H, s), 7.15-8.37 (10H, m)
mass spectrum EIMS, m/z: 372($M^+$)

**Example 14. 1'-(4-methoxybenzyl)-spiroimidazolidine-4,4'-piperidine-3-phenyl-5-on**

(alias: 1-phenyl-8-(4-methoxybenzyl)-1,3,8-triazaspiro[4,5]decane-4-on)

5.00g (21.5mmol) of 1-phenyl-1,3,8-triazaspiro[4,5]decane-4-on (SIGMA) and 2.94g (21.5mmol) of 4-methoxybenzylaldehyde (Tokyo Kasei Kogyo Co., Ltd.) were dissolved in 100ml of 1,2-dichloroethane, and then 11.9ml (215.0mmol) of acetic acid and 6.87g (33.4mmol) of sodium triacetoxy borohydride were added thereto, followed by stirring for 12 hours. Extraction was performed by adding 100ml of chloroform, 30ml of water and 250ml of saturated sodium bicarbonate solution to the reaction solution. After the organic layer was dried with magnesium sulfate anhydrous and the solvent was evaporated, the residue was purified with silica gel column chromatography (methylene chloride : methanol = 20 : 1) to obtain 4.01g of the above compound of interest.
$^1$H NMR ($CDCl_3$, 300MHz) δ 1.71 (2H, brt), 2.58-2.75 (2H, m), 2.80 (4H, brd), 3.53 (2H, s), 3.81 (3H, s), 4.72 (2H, s), 6.84-6.97 (5H, m), 7.24-7.35 (4H, m)
mass spectrum EIMS, m/z: 351 ($M^+$)

**Example 15. 1'-(6-chloro-3-pyridyl)methyl-spiroimidazolidine-4,4'-piperidine-3-phenyl-5-on**

(alias: 1-phenyl-8-[(6-chloro-3-pyridyl)methyl)-1,3,8-triazaspiro[4,5]decane-4-on)

Using 0.15g (0.703mmol) of 1-phenyl-1,3,8-triazaspiro[4,5]decane-4-on (SIGMA) and 0.10g (0.706mmol) of 6-chloropyridine-3-carboaldehyde obtained in Reference example 1, 0.119mg of the above compound of interest was obtained by the same method as in Example 14.
$^1$H NMR ($CDCl_3$, 300MHz) δ 1.73 (2H, brt), 2.61-2.95 (6H, m), 3.57 (2H, s), 4.76 (2H, s), 6.83-6.95 (3H, m), 7.25-7.37 (3H, m), 7.70 (1H, d, J=7.4Hz), 7.79 (1H, s), 8.38 (1H, s)
mass spectrum EIMS, m/z: 356($M^+$)

**Example 16. 1'-(6-methyl-3-pyridyl)methyl-spiroimidazolidine-4,4'-piperidine-3-phenyl-5-on**

(alias: 1-phenyl-8-[(6-methyl-3-pyridyl)methyl]-1,3,8-triazaspiro[4,5]decane-4-on)

Using 6.60g (30.9mmol) of 1-phenyl-1,3,8-triazaspiro[4,5]decane-4-on (SIGMA) and 2.50g (20.5mmol) of 6-methylpyridine-3-carboaldehyde obtained in Reference example 2, 2.70g of the above compound of interest was obtained by the same method as in Example 14.
$^1$H NMR ($CDCl_3$, 300MHz) δ 1.71 (2H, brt), 2.55 (3H, s), 2.62-2.90 (6H, m), 3.56 (2H, s), 4.75 (2H, s), 6.82-6.95 (2H, m), 7.13 (1H, d, J=7.7Hz), 7.24-7.35 (2H, m), 7.59 (1H, d, J=7.7Hz), 7.95 (1H, s), 8.49 (1H, s)
mass spectrum EIMS, m/z: 336($M^+$)

**Example 17. 1'-(3-pyridyl)methyl-spiroimidazolidine-4,4'-piperidine-3-phenyl-5-on**

(alias: 1-phenyl-8-(3-pyridyl)methyl-1,3,8-triazaspiro[4,5]decane-4-on)

Using 0.10g (0.47mmol) of 1-phenyl-1,3,8-triazaspiro[4,5]decane-4-on (SIGMA) and 0.05g (0.47mmol) of 3-pyridylaldehyde (Aldrich), 0.059mg of the above compound of interest was obtained by the same method as in Example 14.

$^1$H NMR ($CDCl_3$, 300MHz) δ 1.72 (2H, brt), 2.63-2.93 (6H, m), 3.60 (2H, s), 4.75 (2H, s), 6.83-6.95 (3H, m), 7.24-7.35 (3H, m), 7.72 (1H, brd), 8.05 (1H, s), 8.51 (1H, d, J=1.6Hz, J=4.8Hz), 8.61 (1H, s)
mass spectrum EIMS, m/z: 322($M^+$)

**Example 18. 1'-(4-bromobenzyl)-spiroimidazolidine-4,4'-piperidine-3-phenyl-5-on**

(alias: 1-phenyl-8-(4-bromobenzyl)-1,3,8-triazaspiro[4,5]decane-4-on)

Using 8.60g (40.3mmol) of 1-phenyl-1,3,8-triazaspiro[4,5]decane-4-on (SIGMA) and 5.00g (27.0mmol) of 4-bromobenzaldehyde (Tokyo Kasei Kogyo Co., Ltd.), 8.12g of the above compound of interest was obtained by the same method as in Example 14.
$^1$H NMR ($CDCl_3$, 300MHz) δ 1.71 (2H, brd), 2.60-2.88 (6H, m), 3.53 (2H, s), 4.74 (2H, s), 6.84-6.97 (3H, m), 7.22-7.37 (4H, m), 7.45 (2H, d, J=8.2Hz), 7.76 (1H, s)
mass spectrum EIMS, m/z: 399, 401($M^+$)

**Example 19. 1-(4-methoxybenzyl)-4-(2-keto-1-benzoimidazolinyl)-piperidine**

Using 0.20g (0.92mmol) of 4-(2-keto-1-benzoimidazolinyl-piperidine (Aldrich) and 0.123g (0.90mmol) of 4-methoxybenzaldehyde (Tokyo Kasei Kogyo Co., Ltd.), 0.133mg of the above compound of interest was obtained by the same method as in Example 14.
$^1$H NMR ($CDCl_3$, 300MHz) δ 1.81 (2H,brt), 2.27 (2H, brt), 2.40-2.59 (2H, m), 3.06 (2H, brd), 3.54 (2H, s), 3.81 (3H, s), 4.32-4.47 (1H, m), 6.85-6.94 (2H, m), 7.01-7.15 (2H,m), 7.24-7.34 (4H, m), 10.4 (1H, brs)
mass spectrum EIMS, m/z: 337($M^+$)

**Example 20. 1-[(6-chloro-3-pyridyl)methyl]-4-(2-keto-1-benzoimidazolinyl)-piperidine**

Using 0.035g (0.161mmol) of 4-(2-keto-1-benzoimidazolinyl)-piperidine (Aldrich) and 0.023g (0.162mmol) of 6-chloropyridine-3-carboaldehyde obtained in Reference example 1, 0.021mg of the above compound of interest was obtained by the same method as in Example 14.
$^1$H NMR ($CDCl_3$, 300MHz) δ 1.83 (2H, brt), 2.22 (2H, brt), 2.41-2.57 (2H, m), 3.01 (2H, brd), 3.56 (2H, s), 4.32-4.45 (1H, m), 7.01-7.17 (3H, m), 7.22-7.29 (1H, m), 7.33 (1H, d, J=8.2Hz), 7.72 (1H, dd, J=2.2Hz, J=8.2Hz), 8.38 (1H, d, J=2.2Hz), 10.0 (1H, s)
mass spectrum EIMS, m/z: 342($M^+$)

**Reference example 12. 7-quinolinealdehyde**

0.67g (6.04mmol) of selenium dioxide (Aldrich) was added to 1.0g (6.98mmol) of 7-methylquinoline (Aldrich), and the mixture was stirred, first, at 150 to 180°C for 6 hours in an oil bath, then at room temperature for 9 hours. 5ml of methylene chloride was added to the reaction mixture and filtrated. The filtrate was evaporated, and the residue was purified with silica gel column chromatography (hexane : ethyl acetate = 4 : 1) to obtain 0.37g of the above compound of interest.
'H NMR ($CDCl_3$, 300MHz) δ 7.56 (1H, dd, J=4.2Hz, J=8.3Hz), 7.95 (1H, d, J=8.3Hz), 8.06 (1H, dd, J=1.6Hz, J=8.3Hz), 8.24 (1H, d, J=8.3Hz), 8.58 (1H, s), 9.05 (1H, dd, J=1.6Hz, J=4.2Hz), 10.3 (1H, s)
mass spectrum EIMS, m/z: 157($M^+$)

**Example 21. 1'-(7-quinolylmethyl)-spiroimidazolidine-4,4'-piperidine-3-phenyl-5-on**

(alias: 1-phenyl-8-(7-quinolylmethyl)-1,3,8-triazaspiro[4,5]decane-4-on)

Using 0.15g (0.954mmol) of 1-phenyl-1,3,8-triazaspiro[4,5]decane-4-on (SIGMA) and 0.11g (0.70mmol) of 7-quinolinealdehyde obtained in Reference example 12, 0.10g of the above compound of interest was obtained by the same method as in Example 14.
$^1$H NMR ($CDCl_3$, 300MHz) δ 1.73 (2H, brt), 2.67-2.80 (2H, m), 2.82-2.98 (4H, m), 3.81 (2H, s), 4.74 (2H, s), 6.63 (1H, s), 6.85-6.98 (3H, m), 7.25-7.42 (3H, m), 7.66 (1H, dd, J=1.6Hz, J=8.3Hz), 7.81 (1H, d, J=8.3Hz), 8.06 (1H, s), 8.15 (1H, d, J=8.3Hz), 8.91 (1H, dd, J=1.6Hz, J=4.2Hz)
mass spectrum EIMS, m/z: 372($M^+$)

**Example 22. 1'-(2-naphtylmethyl)-spiroimidazolidine-4,4'-piperidine-3-phenyl-5-on**

(alias: 1-phenyl-8-(2-naphtylmethyl)-1,3,8-triazaspiro[4,5]decane-4-on)

Using 0.1g (0.47mmol) of 1-phenyl-1,3,8-triazaspiro[4,5]decane-4-on (SIGMA) and 0.073g (0.47mmol) of 2-naphtylaldehyde (Tokyo Kasei Kogyo Co., Ltd.), 0.082g of the above compound of interest was obtained by the same method as in Example 14.
$^1$H NMR ($CDCl_3$, 300MHz) δ 1.72 (2H, brt), 2.63-2.80 (2H, m), 2.81-2.94 (4H, m), 3.75 (2H, s), 4.72 (2H, s), 6.87 (1H, t, J=7.3Hz), 6.93 (2H, d, J=8.3Hz), 7.31 (2H, dd, J=7.3Hz, J=8.3Hz), 7.39-7.50 (2H, m), 7.57 (2H, d, J=7.3Hz), 7.71 (1H, s), 7.75-7.87 (4H, m)
mass spectrum EIMS, m/z: 371($M^+$)

**Example 23. 1'-(4-methylbenzyl)-spiroimidazolidine-4,4'-piperidine-3-phenyl-5-on**

(alias: 1-phenyl-8-(4-methylbenzyl)-1,3,8-triazaspiro[4,5]decane-4-on)

Using 0.1g (0.47mmol) of 1-phenyl-1,3,8-triazaspiro[4,5]decane-4-on (SIGMA) and 0.05ml (0.42mmol) of 4-methylbenzaldehyde (Tokyo Kasei Kogyo Co., Ltd.), 0.115g of the above compound of interest was obtained by the same method as in Example 14.
$^1$H NMR ($CDCl_3$, 300MHz) δ 1.72 (2H, brt), 2.34 (3H, s), 2.60-2.88 (6H, m), 3.55 (2H, s), 4.72 (2H, s), 6.87 (1H, t, J=7.3Hz), 6.93 (2H, d, J=7.9Hz), 7.13 (2H, d, J=7.9Hz), 7.22-7.34 (4H, m), 7.89 (1H, s)
mass spectrum EIMS, m/z:335($M^+$)

**Example 24. 1'-benzyl-7-methoxy(benzo[g]1,3-dihydroisobenzofuran-1-spiro-4'-piperidine)-3-on**

0.410g (1.10mmol) of compound (A) obtained in Example 13 was dissolved in 4.00ml of methylene chloride, then 0.63g (3.31mmol) of p-toluenesulfonic acid 1 hydrate was added thereto, followed by stirring. At 2 days after initiation of the reaction, 1ml of 1M hydrochloric acid was added. At 5 days after initiation of the reaction, extraction was performed by adding 3ml of chloroform and 2ml of saturated sodium bicarbonate solution. After the organic layer was dried with magnesium sulfate anhydrous and the solvent was evaporated, the residue was purified with silica gel column chromatography (methylene chloride : methanol = 20 : 1) to obtain 0.18g of the above compound of interest.
$^1$H NMR ($CDCl_3$, 400MHz) δ 1.77 (2H, brd), 2.69 (4H, m), 2.97 (2H, d, brd), 3.66 (2H, s), 3.97 (3H, s), 7.25-7.45 (7H, m), 7.83 (1H, d, J=8.3Hz), 7.80 (1H, d, J=8.4Hz), 8.12 (1H, d, J=8.4Hz)
mass spectrum EIMS, m/z: 373($M^+$)

**Example 25. 1'-benzyl-7-methoxy(benzo[f]1,3-dihydroisobenzofuran-1-spiro-4'-piperidine)-3-on**

0.100g (0.27mmol) of compound (B) obtained in Example 13 was dissolved in 4.00ml of methylene chloride, then 0.15g (0.81mmol) of p-toluenesulfonic acid 1 hydrate was added thereto, followed by stirring. At 2 days after initiation of the reaction, 1ml of 1M hydrochloric acid was added. At 5 days after initiation of the reaction, extraction was performed by adding 3ml of chloroform and 2ml of saturated sodium bicarbonate solution. After the organic layer was dried with magnesium sulfate anhydrous and the solvent was evaporated, the residue was purified with silica gel column chromatography (methylene chloride : methanol = 20 : 1) to obtain 0.07g of the above compound of interest.
$^1$H NMR ($CDCl_3$, 400MHz) δ 1.81 (2H, brd), 2.30 (2H, dt, J=4.6, 13.0Hz), 2.61 (2H, dt, J=2.4, 13.0Hz), 2.94 (2H, brd), 3.66 (2H, s), 3.97 (3H, s), 7.17-7.42 (7H, m), 7.67 (1H, s), 7.89 (1H, d, J=9.1Hz), 8.55 (1H, s)
mass spectrum EIMS, m/z: 373($M^+$)

**Example 26. 1'-benzyl-7-hydroxy(benzo[g]1,3-dihydroisobenzofuran-1-spiro-4'-piperidine)-3-on**

0.10g (0.267mmol) of the compound obtained in Example 24 was dissolved in 2.0ml of methylene chloride, then 0.54mlof 1.0M boron tribromide was added thereto, followed by stirring for 22 hours in an argon atmosphere. Extraction was performed by adding 2ml of water, 6ml of methylene chloride and 4ml of saturated sodium bicarbonate solution to the reaction mixture. After the organic layer was dried with magnesium sulfate anhydrous and the solvent was evaporated, the residue was purified with silica gel column chromatography (methylene chloride : methanol = 20 : 1) to obtain 0.70g of the above compound of interest.
$^1$H NMR ($CDCl_3$, 400MHz) δ 1.77 (2H, brd), 2.82-2.98 (4H, m), 3.25 (2H, brd), 3.90 (2H, s), 6.91 (1H, s), 7.31-7.53 (6H, m), 7.55 (1H, d, J=9.1Hz), 7.73 (1H, d, J=9.1Hz), 8.45 (1H, d, J=9.1Hz)
mass spectrum EIMS, m/z: 359($M^+$)

**Example 27. 1'-benzyl-2,5-dihydroflo[3,4-b]quinoline-5-spire-4'-piperidine-2-imine**

1.87g (13.3mmol) of 2,2,6,6-tetramethylpiperidine was dissolved in 48ml of tetrahydrofuran anhydrous. The reaction mixture was set to 0°C, and 12ml of 1.14mol/l methyl lithium diethyl ether solution was added thereto, followed by stirring for 30 minutes. The temperature of the reaction mixture was reduced to -78°C, and 2.00g of 3-quinolinecarbonitrile dissolved in 10ml of tetrahydrofuran anhydrous was dropped in the reaction mixture, followed by stirring for 50 minutes. Subsequently, 3.00g (15.9mmol) of 4-benzylpiperidone dissolved in 10ml of tetrahydrofuran anhydrous was added thereto, followed by stirring for 2 hours. The temperature was raised to 0°C, followed by stirring for 1 hour, then further stirring at room temperature for 1 hour. Extraction was performed by adding 50ml of methylene chloride, 10ml of water and 5ml of 2mol/l hydrochloric acid solution to the reaction mixture at 0°C. After the organic layer was dried with magnesium sulfate anhydrous and the solvent was evaporated, the residue was purified with silica gel column chromatography (methylene chloride : methanol = 30 : 1) to obtain 0.451g of the above compound of interest.
$^1$H NMR ($CDCl_3$, 400MHz) δ 1.76 (2H, brd), 2.43-2.64 (4H, m), 2.97 (2H, brd), 3.65 (2H, s), 7.21-7.43 (6H, m), 7.61 (1H, d, J=8.0Hz), 7.83 (1H, d, J=8.0Hz), 7.99 (1H, d, J=8.0Hz), 8.17 (1H, d, J=8.0Hz), 8.70 (1H, brs)
mass spectrum EIMS, m/z: 343($M^+$)

**Example 28. 1'-benzyl-2,5-dihydroflo[3,4-b]quinoline-5-spiro-4'-piperidine-2-on**

0.124g (0.36mmol) of the compound obtained in Example 27 was dissolved in 1.30ml of methylene chloride, then 0.20g (1.05mmol) of p-toluenesulfonic acid l hydrate was added thereto, followed by stirring for 37 hours. Extraction was performed by adding 25ml of methylene chloride and 25ml of saturated sodium bicarbonate solution to the reaction mixture. After the organic layer was dried with magnesium sulfate anhydrous and the solvent was evaporated, the residue was purified with silica gel column chromatography (methylene chloride : methanol = 20 : 1) to obtain 0.121g of the above compound of interest.
$^1$H NMR ($CDCl_3$, 400MHz) δ 1.76 (2H, brd), 2.51-2.64 (4H, m), 2.97 (2H, brd), 3.65 (2H, s), 7.21-7.43 (5H, m), 7.67 (1H, d, J=8.0Hz), 7.91 (1H, d, J=8.0Hz), 8.03 (1H, d, J=8.0Hz), 8.22 (1H, d, J=8.0Hz), 8.73 (1H, s)
mass spectrum EIMS, m/z: 344($M^+$)

**Reference example 13.**

1'-benzyloxycarbonyl-spiro[benzodihydrofuran-1(3H),4'-piperidine]-3-on

1.19g (5.92mmol) of 2-bromobenzoic acid (Tokyo Kasei Kogyo Co., Ltd.) was dissolved in 25ml of tetrahydrofuran anhydrous. The reaction mixture was set to-78°C, and then 8.0ml (11.8mmol) of 1.54mol/l n-butyl lithium *n*-hexane solution was added thereto, followed by stirring for 2 hours. Subsequently, 1.40g (6.00mmol) of 4-benzyloxycarbonylpiperidone dissolved in 5ml of tetrahydrofuran anhydrous was added thereto, followed by stirring for 3 hours. Then, the temperature was raised to 0°C and stirred for 2 hours. Extraction was performed by adding 15ml of water and 10ml of diethyl ether to the reaction mixture at room temperature. The organic layer was refluxed at 40°C for 1 hour, and then was evaporated. Extraction was carried out by adding 15ml of chloroform and 10ml of saturated sodium acid carbonate solution to the residue. After drying with anhydrous magnesium sulfate and evaporation, the residue was purified with silica gel column chromatography (hexane : ethyl acetate = 2 : 1) to obtain 0.34g of the above compound of interest.
$^1$H NMR ($CDCl_3$, 300MHz) δ 1.71 (2H, brd), 2.02-2.18 (2H, m), 3.27-3.43 (2H, m), 4.30 (2H, brs), 5.19 (2H, s), 7.33-7.42 (6H, m), 7.55 (1H, t, J=7.5Hz), 7.69 (1H, dt, J=1.1, 7.5Hz), 7.90 (1H, d, J=7.5Hz)
mass spectrum EIMS, m/z: 337($M^+$)

**Reference example 14.**

spiro[benzodihydrofuran-1(3H),4'-piperidine]-3-on

1'-benzyloxycarbonyl-spiro[benzodihydrofuran-1 (3H),4'-piperidine]-3-on obtained in Reference example 13. was dissolved in 25% hydrogen bromide (acetic acid solution) and stirred for 16 hours. Extraction was performed by adding 10ml of water, 20ml of chloroform and 50ml of saturated sodium bicarbonate solution thereto. After the organic layer was dried with magnesium sulfate anhydrous and the solvent was evaporated, the residue was purified with silica gel column chromatography (methylene chloride : methanol : ammonia water = 100 : 10 : 1) to obtain 0.02g of the above compound of interest.
$^1$H NMR ($CDCl_3$, 300MHz) δ 1.71 (2H, d, J=12.3Hz), 1.90 (1H, brs) 2.04-2.17 (2H, m), 3.05-3.25 (4H, m), 7.43 (1H, t, J=7.6Hz), 7.53 (1H, dt, J=1.0, 7.6Hz), 7.68 (1H, dt, J=1.0, 7.6Hz), 7.90 (1H, d, J=7.6Hz)

mass spectrum EIMS, m/z: 203($M^+$)

**Reference example 15.**

1'-benzyl-spiro[benzodihydrofuran-1(3H),4'-piperidine)-3-on

0.020g (0.086mmol) of spiro[benzodihydrofuran-1(3H),4'-piperidine]-3-on obtained in Reference example 14. and 0.011ml (0.106mmol) of benzaldehyde were dissolved in 0.4ml of 1,2-dichloroethane, then 0.054ml (0.984mmol) of acetic acid and 0.031g (0.15mmol) of sodium triacetoxy borohydride were added thereto, followed by stirring for 3 hours. Extraction was performed by adding 3ml of chloroform, 1ml of water and 5ml of saturated sodium bicarbonate solution to the reaction solution. After the organic layer was dried with magnesium sulfate anhydrous and the solvent was evaporated, the residue was purified with silica gel column chromatography (methylene chloride : methanol = 10 : 1) to obtain 0.016g of the above compound of interest.
mass spectrum EIMS, m/z: 293($M^+$)

Up to this point, synthesis examples of the compounds of the present invention are discribed. Hereafter, the compounds disclosed in Examples are classified into some groups to describe each of the chemical structures.

Compounds classified into the compounds of general formula (I) include the compounds shown in the following formulas (IV), (V) and (IX):

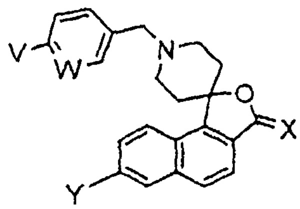

(IV)

| Examples | V | W | X | Y |
|---|---|---|---|---|
| 1 | Cl | N | NH | H |
| 2 | Cl | N | O | H |
| 4 | F | CH | NH | H |
| 5 | Cl | CH | NH | H |
| 6 | $OCH_3$ | CH | NH | H |
| 7 | F | CH | O | H |
| 8 | Cl | CH | O | H |
| 9 | OCH3 | CH | O | H |
| 10 | H | CH | NH | H |
| 11 | H | CH | O | H |
| 13 (A) | H | CH | NH | OCH3 |
| 24 | H | CH | O | OCH3 |
| 26 | H | CH | O | OH |

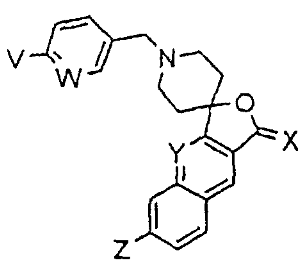

(V)

| Examples | V | W | X | Y | Z |
|---|---|---|---|---|---|
| 3 | Cl | N | O | CH | H |
| 13 (B) | H | CH | NH | CH | OCH3 |
| 25 | H | CH | O | CH | OCH3 |
| 27 | H | CH | NH | N | H |
| 28 | H | CH | O | N | H |

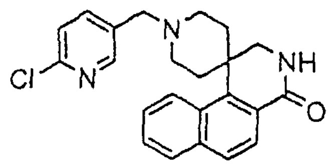

(IX)

Compounds classified into the compounds of general formula (II) include the compounds shown in the following formulas (VI) and (VII):

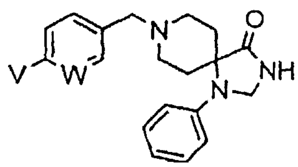

(VI)

| Examples | V | W |
|---|---|---|
| 14 | $OCH_3$ | CH |
| 15 | Cl | N |
| 16 | $CH_3$ | N |
| 17 | H | N |
| 18 | Br | CH |
| 23 | $CH_3$ | CH |

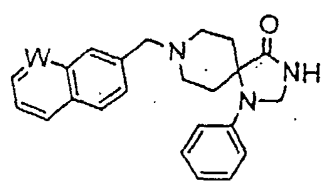

(VII)

| Examples | W |
|---|---|
| 21 | N |
| 22 | CH |

Furthermore, compounds classified into the compounds of general formula (III) include the compound shown in the following formula (VIII):

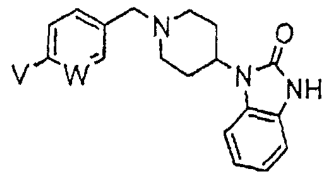

(VIII)

| Examples | V | W |
|---|---|---|
| 19 | OCH3 | CH |
| 20 | Cl | N |

Test example 1. Binding affinity with a μ opioid receptor

A membrane fraction obtained from rat forebrain was prepared as a membrane fraction of a μ opioid receptor. First, rat forebrain was homogenized with 10 × volume of 0.32M sucrose solution, and the obtained homogenate was centrifuged at 900 × g for 10 minutes. Then, the supernatant was centrifuged at 11,500 × g for 20 minutes to obtain a deposit. The pellet was resuspended in an assay buffer (50mM Tris-HCl, pH7.4) and centrifuged again, and the finally obtained membrane fraction was defined as a membrane fraction of a μ opioid receptor.

A binding experiment was carried out with the obtained membrane fraction and a radioactive ligand [$^3$H]-DAMGO. In the presence of a test compound, the membrane fraction and 1nM final concentration of [$^3$H]-DAMGO were added and incubated at 25°C for 90 minutes. The reaction was terminated by rapid filtration with a GF/B filter, then washed with 5ml of assay buffer. Radioactivity was determined with a liquid scintillation counter. Nonspecific binding was determined with 1 μM DAMGO, and specific binding was calculated as the difference between the two values. After the $IC_{50}$ value of each compound was determined by a nonlinear method of least squares regression analysis, Ki value was calculated using Cheng and Prusoff's formula.

As a consequence, it became clear that the compounds of the present invention have a strong affinity with a μ opioid receptor, in that the Ki value for a μ receptor affinity of the compound of Reference example 15 was more than 1,000nM, whereas the Ki value of the compound of the present invention was less than 50nM.

Test example 2. Binding affinity with a dopamine D2 receptor

A membrane fraction obtained from rat corpus striatum was prepared as a membrane fraction of a dopamine D2 receptor. To prepare a sample, first, rat corpus striatum was homogenized with 10 × volume of 0.32M sucrose solution, and then the obtained homogenate was centrifuged at 900 × g for 10 minutes. Then, the supernatant was centrifuged at 11,500 × g for 20 minutes to obtain a deposit. The pellet was resuspended in a dopamine D2 assay buffer (50mM Tris-HCl buffer containing 120mM NaCl, 5mM KCl, 1mM $MgCl_2$ and 1mM $CaCl_2$, pH7.4) and centrifuged again, and the finally obtained pellet was defined as a membrane fraction of a dopamine D2 receptor.

A binding experiment was carried out with the obtained membrane fraction and a radioactive ligand [$^3$H]-spiperone. In the presence of a test compound, the membrane fraction and 0.1nM final concentration of [$^3$H]-spiperone were added and incubated at 37°C for 30 minutes. The reaction was terminated by rapid filtration with a GF/B filter, then washed with 5ml of assay buffer (50mM Tris-HCl, pH7.4). Radioactivity was determined with a liquid scintillation counter. Nonspecific binding was determined with 10 μ M sulpiride, and specific binding was calculated as the difference between the two values. After the $IC_{50}$ value of each compound was determined by a nonlinear method of least squares regression analysis, Ki value was calculated using Cheng and Prusoff's formula.

The results showing the binding affinity of the compounds of the present invention with both a μ opioid receptor and a dopamine D2 receptor in the above Test examples 1 and 2 are presented in the following Table 1.

As a consequence, since the ratio between a μ opioid receptor agonist activity and a dopamine D2 receptor antagonist activity of the compound of the present invention are within the range from 1 : 1 to 1 : 150, it can be said that the compound of the present invention is useful as pain control agent, regulating side effects.

Table 1.

| Test compounds | | μ Ki value(nM) | D2Ki value(nM) | μ Ki value : D2Ki value |
|---|---|---|---|---|
| Compounds of the present invention | Example 1 | 10 | 230 | 1:23 |
| | Example 2 | 3.7 | 532 | 1:144 |
| | Example 3 | 37 | 1588 | 1:43 |
| | Example 11 | 41 | 177 | 1:4.3 |
| | Example 12 | 9.8 | 252 | 1:26 |
| | Example 14 | 2.7 | 20 | 1:7.4 |
| | Example 15 | 0.4 | 3.6 | 1:9 |
| | Example 16 | 0.6 | 17 | 1:28 |
| | Example 17 | 15 | 457 | 1:30 |
| | Example 18 | 3.4 | 5.7 | 1:1.7 |
| | Example 19 | 10 | 60 | 1:6 |
| | Example 20 | 5.4 | 76 | 1:14 |
| | Example 23 | 4.2 | 52 | 1:12.4 |
| Reference example 15 | | >1000 | >1000 | ------ |
| Morphine | | 1.0 | >1000 | ------ |

Test example 3. Analgetic action determined by hot plate test

This test is a common method for evaluating an analgetic action against severe pain, and it is known that a compound having μ opioid receptor agonist activity reacts well in this test. This test was carried out according to the method of Narita *et al*. (*Jpn J Pharmacol*. 1993; 62:15-24). That is to say, ddY male mice (body weight: 28 to 33g) were used and a remedy was intraperitoneally administered thereto. At 30 minutes after administration, the mice were put on a 52°C hot plate and the latency to paw-tap, paw-lick or an attempt to by jumping was measured. The above latency before the administration of drug was previously measured at least 1 hour before the experiment, so that any mice ineligible for this experiment, whose reaction period went beyond 30 seconds, could be determined and eliminated. In addition, in order to avoid the thermal burn of tissues, the longest latency was set at 60 seconds. Analgetic effect was calculated by the following formula.

$$\text{Analgetic effect (\%)} = (\text{test latency} - \text{control latency}) / (60 \text{ seconds} - \text{control latency}) \times 100$$

Then, a sigmoid curve was determined from dose response by a method of least squares, and 50% effective dose ($ED_{50}$ value) was calculated. The $ED_{50}$ values in respect of the analgetic effect of the compounds of the present invention and morphine are shown in the following Table 2.

Table 2.

| Test substances | | $ED_{50}$ value (mg/kg) regarding analgetic effect |
|---|---|---|
| Compounds of the present invention | Example 14 | 2.99 |
| | Example 15 | 1.79 |
| | Example 16 | 1.98 |
| | Example 17 | 15.1 |
| | Example 18 | 7.76 |
| | Example 19 | 7.8 |
| | Example 23 | 7.23 |

Table 2. (continued)

| Test substances | $ED_{50}$ value (mg/kg) regarding analgetic effect |
|---|---|
| Morphine | 13.72 |

The compounds of the present invention have equivalent or stronger analgetic effect than morphine, and from the results of this test and Test example 1., the compounds of the present invention are assumed to have a μ opioid receptor agonist activity.

Test example 4. Anti-dopamine action against apomorphine-induced climbing behavior

The administration of dopamine receptor agonists, apomorphine induces a continual climbing behavior in mice. A test for competitive action against this effect is generally used as a method for detecting the dopamine receptor antagonist. A mouse was put into a cylindrical wire gauze (10 cm diameter × 14cm height) and allowed to habituate itself with the circumstances for about 1 hour. Apomorphine (2mg/kg, s.c.) was administered to the mouse at 10 minutes after the administration of the test drug, and from 20 minutes after the administration of apomorphine, its climbing behavior (which means the period during which all four legs of the mouse have left from the ground) was measured for 1 minute. Furthermore, a sigmoid curve was determined from dose response by a method of least squares, and $ED_{50}$ value was calculated. The $ED_{50}$ values in respect of the dopamine-antagonising effect of the compounds of the present invention are shown in the following Table 3.

Table 3.

| Test substances | | $ED_{50}$ value (mg/kg) regarding anti-dopamine action | Wall climbing behavior |
|---|---|---|---|
| Apomorphine + compounds of the present invention | Example 14 | 2.22 | inhibitory effect |
| | Example 15 | 1.22 | inhibitory effect |
| | Example 16 | 11.9 | inhibitory effect |
| | Example 18 | 1.05 | inhibitory effect |
| | Example 23 | 9.5 | inhibitory effect |
| Control (only Apomorphine) | | | inducing |

All of the compounds of the present invention showed inhibitory effects against the mouse's climbing behavior induced by apomorphine. From these results, it was shown that the compounds of the present invention have a dopamine receptor antagonist activity *in vivo*. Together with all results of Test examples 1 to 3, it was shown that the compounds of the present invention have both μ opioid receptor agonist activity and dopamine D2 receptor antagonist activity *in vivo*.

Test example 5. Influence on locomotor activity

It is generally known that the administration of compounds inducing psychological dependence such as morphine to a mouse promotes its locomotion. This effect is considered to be caused by increase of dopamine release promoting action by means of a μ receptor. Thus, the influence of each compound on the locomotor activity was determined.

Mice were put into a test cage (23 × 33 × 12.5cm) and allowed to habituate with the cage. Then, test substances were intraperitoneally administered to the mice, their locomotor activity was measured for 2 hours, using an infrared sensor.

Morphine showed a strong locomotor activity promoting effect, whereas the compounds of the present invention (Examples 14 to 18, 22 and 23) showed no effect on locomotor activity. Thus, dopamine releasing effects mediated through activation of μ opioid receptors of compounds were marked in the compounds of the present invention having both μ opioid receptor agonist activity and dopamine D2 receptor antagonist activity. Therefore, it is assumed that the compounds of the present invention can regulate side effects such as psychological dependence, nausea, hallucination and derangement, which are considered to be caused by a dopamine releasing effects through μ opioid receptors.

Test example 6. Conditioned position palatability test

Sprague-Dawley rats (170 to 210g) were used. A color-coded shuttle box (30cm width × 60cm length × 30cm height) which was divided into two compartments (a textured white floor and a flat, smooth black floor) was used as an experiment device.

After a test compound or a saline was intraperitoneally administered to a rat (with the exception that morphine was administered subcutaneously), the rat was put into one section for 50 minutes to be conditioned, and on the following day, after a different remedy was administered, the rat was put into another section to be conditioned in the same manner. In order to avoid bias caused by administration order or combination of administration and section, a counterbalance method was adopted. The rat was put into each section immediately after the administration of test remedies. After conditioning on the $6^{th}$ (or $8^{th}$) day, a test was performed on the $7^{th}$ (or $9^{th}$) day to analyze position palatability to the section conditioned by test compound administration.

This position palatability test was carried out as follows: A rat, to which neither a test compound nor saline was administered, was put on a platform installed in the center of the box, and then the period during which the rat stayed in each section was measured from the moment the rat came down from the platform for 15 minutes. The difference between the period during which the rat stayed in the section corresponding to test compound administration, and the period during which the rat stayed in the section corresponding to administration of saline was calculated.

The results are shown in Figures 1 and 2. This test is generally used as a simple method for determining psychological dependence, and where the obtained value is positive, it means that position palatability to the compartment for test compound-administration exists, that a reward exists toward a test compound used for conditioning, in other words, that the used drug has psychological dependence. On the other hand, where the value is negative, it means that place aversion exists, and that is, a test compound used for conditioning has an aversive effect. From the above results, it clearly showed that the compounds of Examples 14 and 16 expressed neither psychological dependence nor aversive effects, while morphine showed a strong rewarding effect.

Industrial Applicability of the Invention

According to the present invention, it becomes possible to provide a novel pain control agent, which shows, with a single agent, a potent analgetic effect at least equivalent to that of morphine, causing no psychological dependence and regulating side effects.

In particular, a novel compound shown in general formula (I) and a pharmacologically acceptable salt thereof have both μ opioid receptor agonist activity and dopamine D2 receptor antagonist activity, and so is useful as pain control agent with regulated side effects.

All publications, patents and patent applications cited herein are incorporated herein by reference in their entirety.

## Claims

1. A pain control agent, which contains, as an active ingredient, a compound having both μ opioid receptor agonist activity and dopamine D2 receptor antagonist activity.

2. The pain control agent according to claim 1, wherein the ratio between the μ opioid receptor agonist activity and dopamine D2 receptor antagonist activity is between 1 : 1 to 1: 150.

3. The pain control agent according to 1 or 2, wherein the active ingredient is a compound selected from a group consisting of the following general formulas (I) to (III) or a pharmacologically acceptable salt thereof:

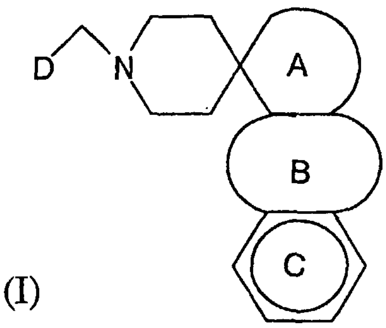

(I)

wherein

ring A represents a saturated 5- or 6-membered ring, which may be substituted and may comprise a heteroatom (s) selected from S, N and O,
ring B represents a 5- or 6-membered aromatic ring, which may comprise a heteroatom selected from N and O,
ring C represents a benzene ring or a pyridine ring, which may be substituted, and
ring D represents an aromatic ring, which may be substituted and may comprise a heteroatom(s) selected from S, N and O;

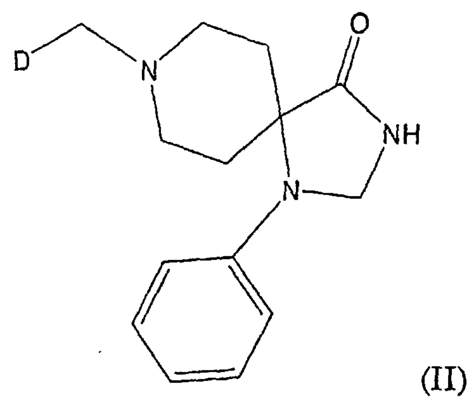

(II)

wherein
D represents an aromatic ring, which may be substituted and may comprise a heteroatom(s) selected from S, N and O; and

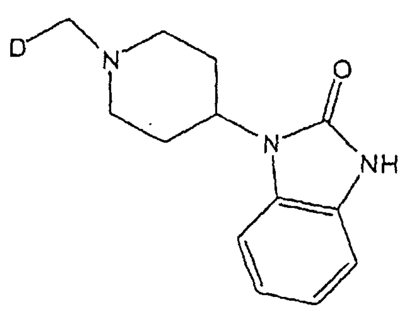

(III)

wherein
D represents an aromatic ring, which may be substituted and may comprise a heteroatom(s) selected from S, N and O.

**4.** A compound shown in the following general formula (I) or pharmacologically acceptable salts thereof:

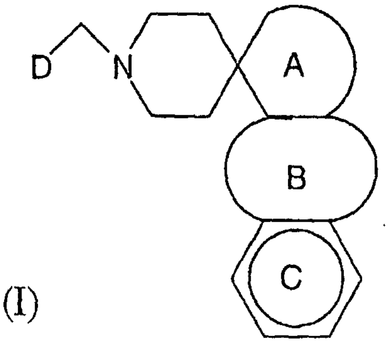

(I)

wherein

ring A represents a saturated 5- or 6-membered ring, which may be substituted and may comprise a heteroatom(s) selected from S, N and O,
ring B represents a 5- or 6-membered aromatic ring, which may comprise a heteroatom selected from N and O,
ring C represents a benzene ring or a pyridine ring, which may be substituted, and
ring D represents an aromatic ring, which may be substituted and may comprise a heteroatom(s) selected from S, N and O.

**5.** The compound or pharmacologically acceptable salts thereof according to claim 4, wherein

ring A is shown in any one of the following general formulas (a) to (e):

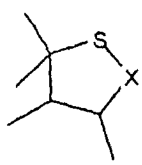

(a)

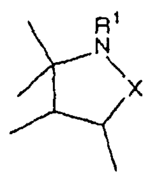

(b)

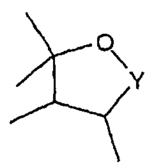

(c)

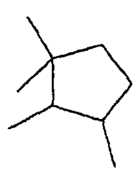

(d)

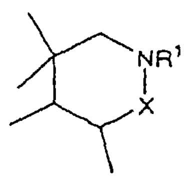

(e)

wherein $R^1$ is H or lower alkyl,

X is $CH_2$ or C=O, and
Y is $CH_2$, C=O or C=NH;

ring B is shown in any one of the following general formulas (f) to (h):

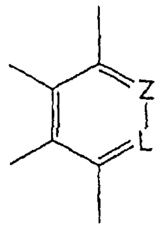
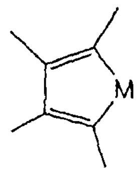
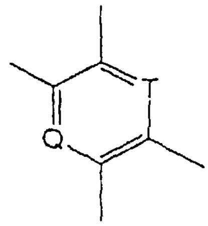

(f) (g) (h)

wherein

either one of Z and L is N and the other is CH, or both are CH,
M is NH or O, and
either one of Q and T is N and the other is CH, or both are CH; and

D is shown in any one of the following general formulas (i) to (n):

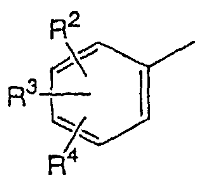
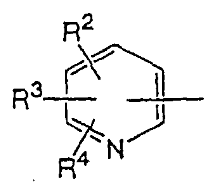
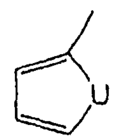

(i) (j) (k)

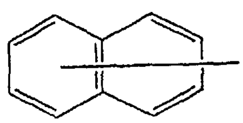
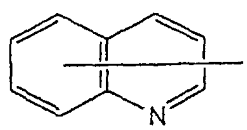
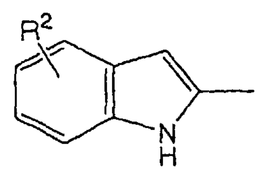

(l) (m) (n)

wherein

each of $R^2$, $R^3$ and $R^4$ represents independently hydrogen, lower alkyl, lower alkoxy, lower alkylthio, lower alkoxycarbonyl, cyano, halogen, hydroxy, carbamoyl, nitro or amino, and
U represents S, O or $NR^5$ ($R^5$ is H or lower alkyl).

**6.** The compound or pharmacologically acceptable salt thereof according to claim 5, wherein ring C is benzene.

**7.** The compound or pharmacologically acceptable salt thereof according to claim 6, wherein

ring A is (c), wherein Y is C=O or C=NH;
ring B is (f), wherein both of Z and L are CH; and
D is (i) or (j), wherein each of $R^2$, $R^3$ and $R^4$ is independently chlorine, fluorine, hydrogen or methoxy.

**8.** A compound shown in the following general formula (IV) or pharmacologically acceptable salt thereof:

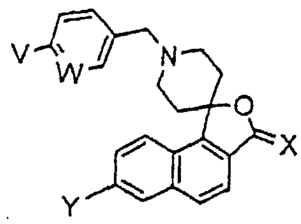

(IV)

wherein V is hydrogen, chlorine, fluorine or methoxy; W is nitrogen or CH; X is NH or O; and Y is hydrogen, hydroxyl or methoxy.

**9.** A compound shown in the following general formula (V) or pharmacologically acceptable salt thereof:

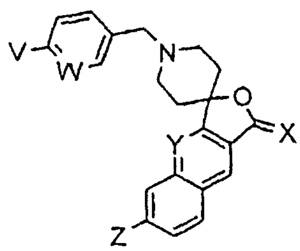

(V)

wherein V is hydrogen or chlorine; W is nitrogen or CH; X is NH or O; Y is CH or nitrogen, and Z is hydrogen or methoxy.

**10.** A compound shown in the following general formula (VI) or pharmacologically acceptable salt thereof:

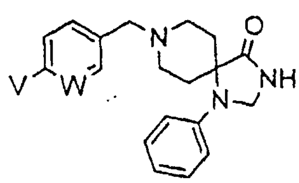

(VI)

wherein V is hydrogen, chlorine, bromine, methyl or methoxy; W is nitrogen or CH.

**11.** A compound shown in the following general formula (VII) or pharmacologically acceptable salt thereof:

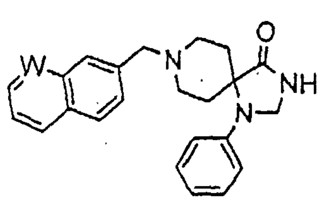

(VII)

wherein W is nitrogen or CH.

**12.** A compound shown in the following general formula (VIII) or pharmacologically acceptable salt thereof:

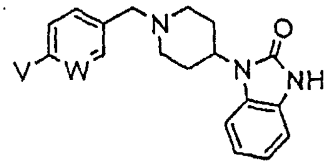

(VIII)

wherein V is chlorine or methoxy, and W is nitrogen or CH.

**13.** A compound shown in the following general formula (IX) or pharmacologically acceptable salt thereof:

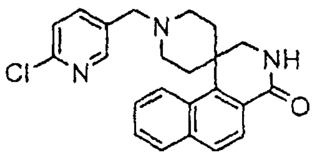

(IX)

**14.** A remedy containing the compound or pharmacologically acceptable salt thereof according to any one of claims 4 to 13.

**15.** Pain control agent containing the compound or pharmacologically acceptable salt thereof according to any one of claims 4 to 13.

Fig. 1

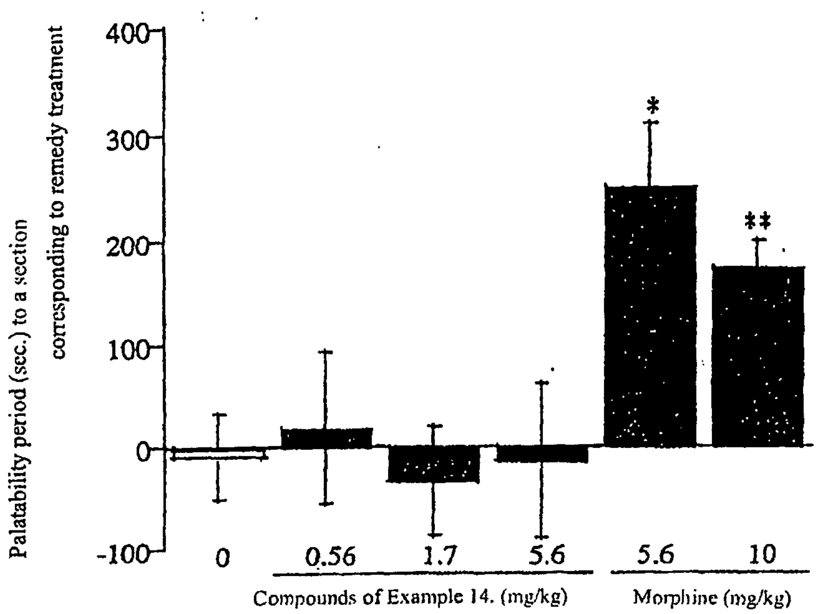


When compared with a solvent administration group (0), each of * and ** acts to significantly increase palatability, showing P<0.05 and P<0.01, respectively.

Fig. 2

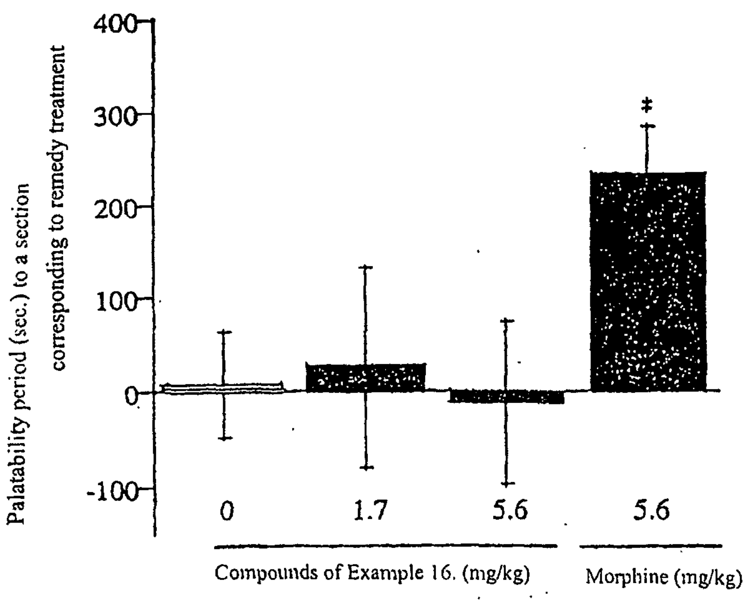


When compared with a solvent administration group (0), * acts to significantly increase palatability, showing P<0.05.

## INTERNATIONAL SEARCH REPORT

International application No.
PCT/JP99/07191

A. CLASSIFICATION OF SUBJECT MATTER
Int.Cl[7] A61K45/00, A61K31/438, A61K31/4409, C07D401/14, C07D401/04, A61P25/04, C07D491/107, C07D471/10

According to International Patent Classification (IPC) or to both national classification and IPC

B. FIELDS SEARCHED

Minimum documentation searched (classification system followed by classification symbols)
Int.Cl[7] A61K45/00, A61K31/438, A61K31/4409, C07D401/14, C07D401/04, A61P25/04, C07D491/107, C07D471/10

Documentation searched other than minimum documentation to the extent that such documents are included in the fields searched
Jitsuyo Shinan Koho 1940-1992 Toroku Jitsuyo Shinan Koho 1994-1996
Kokai Jitsuyo Shinan Koho 1971-1992 Jitsuyo Shinan Toroku Koho 1996-1999

Electronic data base consulted during the international search (name of data base and, where practicable, search terms used)
CA(STN),REGISTRY(STN),MEDLINE(STN),EMBASE(STN)

C. DOCUMENTS CONSIDERED TO BE RELEVANT

| Category* | Citation of document, with indication, where appropriate, of the relevant passages | Relevant to claim No. |
|---|---|---|
| X | Swain, Henry,"Annual Report:evaluation of new compounds for opioid activity",Chemical Abstracts,Vol.93, Abstract No. 107010(1980), Full test | 3 |
| X | Rafael Maldonado,"Absence of opiate rewarding effects in mice lacking dopamine D2 receptors", Nature, Vol.388, No.6642 (1997), P.586-589, Full text, especially, P.587, right column, lines 12~15, ABSTRACT, lines 15~17 | 1,2 |
| X<br>A | Josee E Leysen,"[3H]Sufentanil, a Superior Ligand for μ-Opiate Receptors:Binding Properties and Regional Distribution in Rat Brain and Spinal Cord",European Journal of Pharmacology,Vol.87,No.2-3(1983)P.209-225 Full text, especially, P.216, Compound Nos. R6582,R4782; Table 2B; Fig.6 | 3,12,14,15<br>5-11,13 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 March, 2000 (10.03.00) | 21 March, 2000 (21.03.00) |
| Name and mailing address of the ISA/ Japanese Patent Office | Authorized officer |
| Facsimile No. | Telephone No. |

Form PCT/ISA/210 (second sheet) (July 1992)

## INTERNATIONAL SEARCH REPORT

International application No.
PCT/JP99/07191

**Box I Observations where certain claims were found unsearchable (Continuation of item 1 of first sheet)**

This international search report has not been established in respect of certain claims under Article 17(2)(a) for the following reasons:

1. ☐ Claims Nos.:
because they relate to subject matter not required to be searched by this Authority, namely:

2. ☒ Claims Nos.: 3,4,14,15
because they relate to parts of the international application that do not comply with the prescribed requirements to such an extent that no meaningful international search can be carried out, specifically:

In compounds represented by the chemical structural formula wherein the rings A, B and C are fused together as claimed in claims 3 and 4, the binding manners of these plural fused rings are not clearly described. Therefore, it is unclear which compounds fall within this scope. Accordingly, the International Search is not effected on the parts of the compounds represented by these fused rings. The same applies to claims with the citation of claim 4.

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)